# EUROPEAN PATENT APPLICATION

(11) **EP 4 129 295 A1**
(43) Date of publication of application: **08.02.2023**
(21) Application number: 21750667.4
(22) Date of filing: 05.02.2021
(51) Int. Cl.: A61K 31/5377, A61P 35/00

(54) **PHARMACEUTICAL COMPOSITION FOR PREVENTION OR TREATMENT OF CANCERS ASSOCIATED WITH KRAS MUTATION**

(30) Priority: 06.02.2020 KR 20200014249
(71) Applicant: Wellmarker Bio Co., Ltd., Seoul 05855 (KR)
(72) Inventor: SHIN, Jae-Sik, Seoul 05855 (KR); LEE, Jung-Eun, Seoul 05855 (KR); JEONG, Joon-Yee, Seoul 05855 (KR); LEE, Min-Ki, Seoul 05855 (KR); KIM, Hyo-Jin, Seoul 05855 (KR); CHOI, Soon-Jin, Seoul 05855 (KR); GO, Ji-Hyun, Seoul 05855 (KR)
(74) Representative: HGF
(86) International application number: PCT/KR2021/001566
(87) International publication number: WO 2021/158071

(57) **Abstract**

The present invention relates to a pharmaceutical composition for preventing or treating cancer, which is associated with a KRAS mutation and exhibits resistance to an EGFR-targeted therapeutic agent. The pharmaceutical composition can be useful for treatment of patients with cancer which has the KRAS mutation and exhibits resistance to cetuximab that is an anticancer therapeutic agent.

## Description

### Technical Field

The present invention relates to a pharmaceutical composition for preventing or treating cancer associated with a KRAS mutation. Specifically, the present invention relates to a pharmaceutical composition comprising a compound for preventing or treating cancer, which is associated with a KRAS mutation and exhibits resistance to an EGFR-targeted therapeutic agent.

### Background Art

A V-Ki-ras2 Kirsten rat sarcoma viral oncogene homolog (KRAS) gene is one of several genes associated with the development of non-small cell lung cancer, colorectal cancer, pancreatic cancer, and the like, and has been recently considered crucial to determine the effectiveness of anticancer therapy for patients who exhibit resistance to an anticancer drug such as cetuximab.

Cetuximab is a monoclonal antibody that targets an epidermal growth factor receptor (EGFR), and specifically binds to the EGFR on the cell surface so that proliferation of cancer cells is inhibited. In particular, since the EGFR is overexpressed in cancer cells of metastatic colorectal cancer, metastatic non-small cell lung cancer, and metastatic head and neck cancer, cetuximab is mainly used for treatment of such diseases.

However, cetuximab cannot be used for patients who exhibit resistance to an anticancer drug, and the resistance to such an anticancer drug occurs for several reasons. Typically, cancer cells with a mutation in certain protein such as KRAS, which is located in the EGFR signaling system, exhibit resistance to cetuximab. Thus, drug response and survival in patients who exhibit resistance to an anticancer drug may be determined by identifying a mutation of the KRAS gene. In addition, even for colorectal cancer patients who carry a normal KRAS gene, if continuously treated with cetuximab, 60% to 80% of the patients undergo a mutation in the KRAS gene.

Therefore, to effectively treat cancer, a new anticancer drug, which is also applicable to cancer including a KRAS mutation which exhibits resistance to conventional anticancer drugs, is being required.

### [PRIOR ART DOCUMENTS]

(Non-patent document 1) KRAS mutation status is predictive of response to cetuximab therapy in colorectal cancer, Cancer Res., (2006) 66(8), 3992-3995
(Non-patent document 2) KRAS Mutations As an Independent Prognostic Factor in Patients With Advanced Colorectal Cancer Treated With Cetuximab, J Clin Oncol., (2008), 26, 374-379
(Non-patent document 3) Genomic characterization of intrinsic and acquired resistance to cetuximab in colorectal cancer patients, Sci Rep., (2019), 9, 15365-15377
(Non-patent document 4) Mechanisms of resistance to anti-EGFR therapy in colorectal cancer. Oncotarget. (2017), 8(3), 3980-4000.

### Disclosure of Invention

### Technical Problem

An object of the present invention is to provide a pharmaceutical composition for preventing or treating cancer associated with a KRAS mutation, the composition comrising, as an active ingredient, a compound capable of effectively killing cancer cells having a KRAS mutation which exhibit resistance to an EGFR-targeted therapeutic agent, or a pharmaceutically acceptable salt thereof.

Another object of the present invention is to provide a method for identifying the KRAS mutation and providing information on an anticancer therapeutic agent suitable therefor.

### Solution to Problem

In order to achieve the object, the present invention provides a pharmaceutical composition for preventing or treating cancer associated with a V-Ki-ras2 Kirsten rat sarcoma viral oncogene homolog (KRAS) mutation, the composition comprising, as an active ingredient, a compound represented by Formula 1 or Formula 2 below, or a pharmaceutically acceptable salt thereof, wherein the cancer exhibits resistance to an epidermal growth factor receptor (EGFR)-targeted therapeutic agent, and the KRAS mutation is substitution of glycine, an amino acid residue, at position 13 in an amino acid sequence of SEQ ID NO: 1 with aspartic acid:

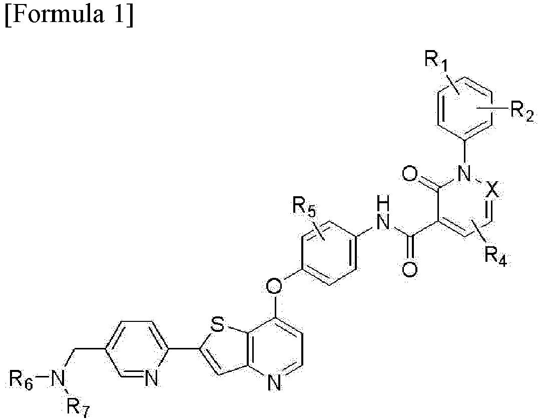

In Formula 1 above, R₁ to R₇, and X are the same as defined in the detailed description which will be described later.

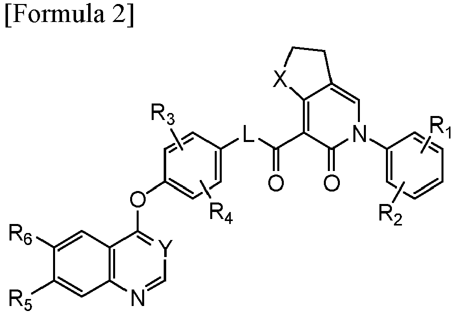

In Formula 2 above, R₁ to R₆, L, X, and Y are the same as defined in the detailed description which will be described later.

In order to achieve another object, the present invention provides a method for providing information on an anticancer therapeutic agent, the method comprising:
identifying a KRAS mutation in an individual who exhibits resistance to an EGFR-targeted therapeutic agent; and providing information that the compound represented by Formula 1 or Formula 2 as defined above or a pharmaceutically acceptable salt thereof is suitable for anticancer therapy when a KRAS mutation, in which glycine, an amino acid residue, at position 13 in the amino acid sequence of SEQ ID NO: 1 is substituted with aspartic acid, is found.

### Advantageous Effects of Invention

The pharmaceutical composition for preventing or treating cancer according to the present invention is applicable to patients with cancer having a KRAS mutation. In particular, the pharmaceutical composition may be useful for treatment of patients with cancer which has a KRAS G13D mutation and exhibits resistance to cetuximab that is conventionally used for anticancer therapy.

In addition, the method for providing information on an anticancer therapeutic agent according to the present invention may identify the KRAS mutation and provide information on an anticancer therapeutic agent suitable therefor.

### Brief Description of Drawings

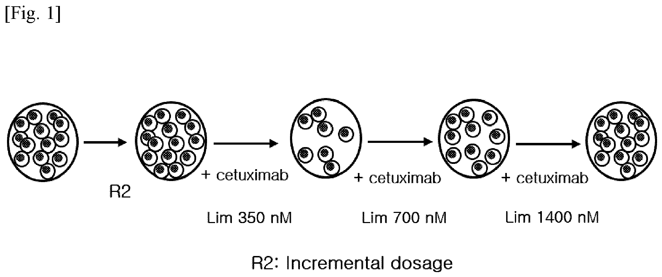

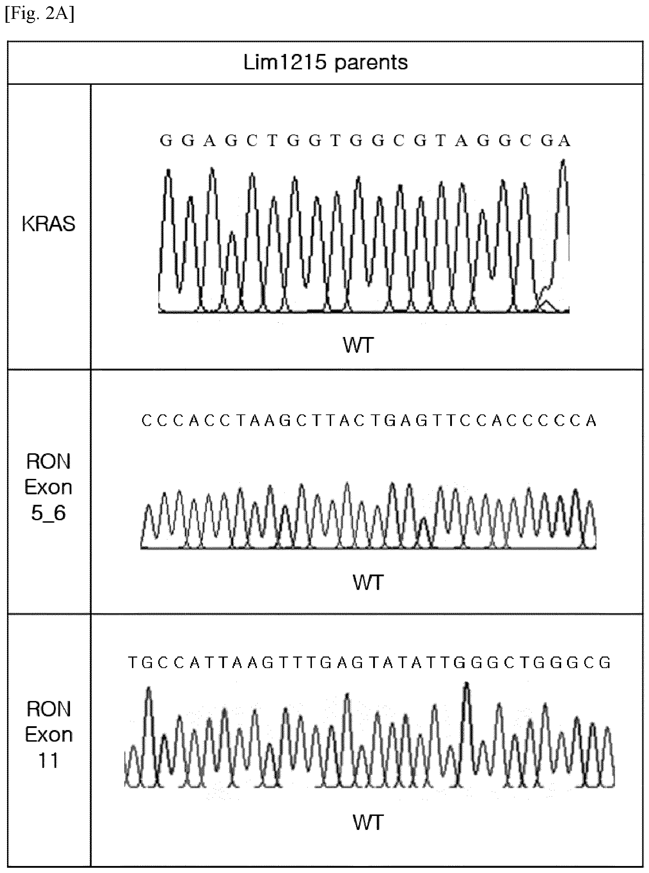

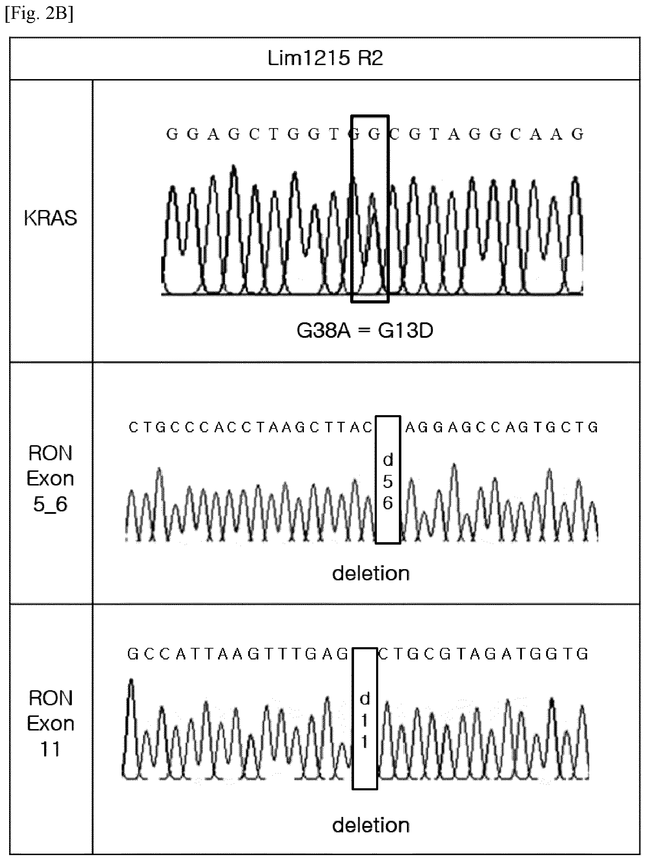

FIG. 1 illustrates a schematic diagram showing a process of obtaining cell lines exhibiting resistance to cetuximab from colorectal cancer cell line LIM1215.
Each of FIGS. 2A and 2B illustrates results obtained by analyzing whether RON and KRAS mutations have occurred in a cell line sensitive to cetuximab, and a cell line exhibiting resistance to cetuximab from the colorectal cancer cell line LIM1215.

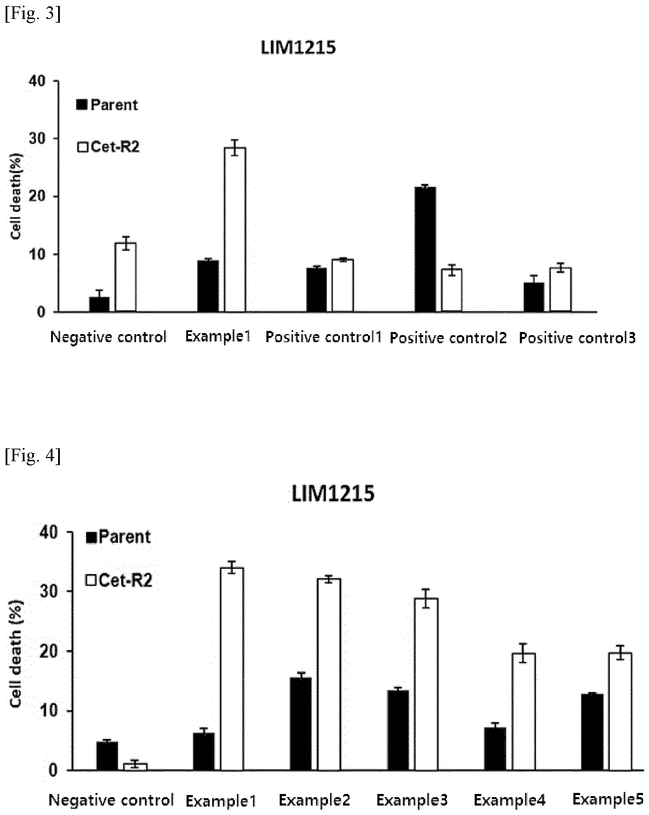

FIG. 3 shows results obtained by treating each of the cell line sensitive to cetuximab and the cell line exhibiting resistance to cetuximab, from the colorectal cancer cell line LIM1215, with a compound of a negative control (DMSO), Example 1, positive control 1, positive control 2, or positive control 3, and comparing cell death rates thereof.
FIG. 4 shows results obtained by treating each of the cell line sensitive to cetuximab and the cell line exhibiting resistance to cetuximab, from the colorectal cancer cell line LIM1215, with a compound of a negative control (DMSO), Example 1, Example 2, Example 3, Example 4, or Example 5, and comparing cell death rates thereof.

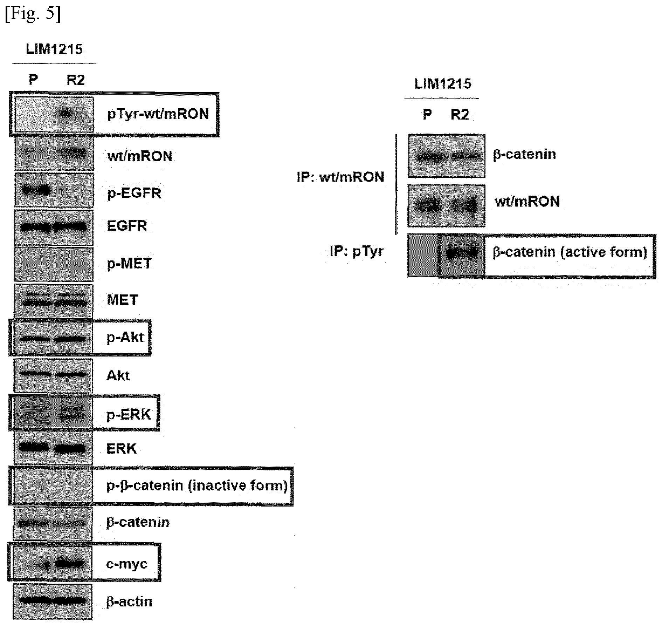

FIG. 5 shows results obtained by performing the western blotting to analyze the action mechanism of the compound of Example 1 against the colorectal cancer cell line LIM1215 having a KRAS G13D mutation and exhibiting resistance to cetuximab.

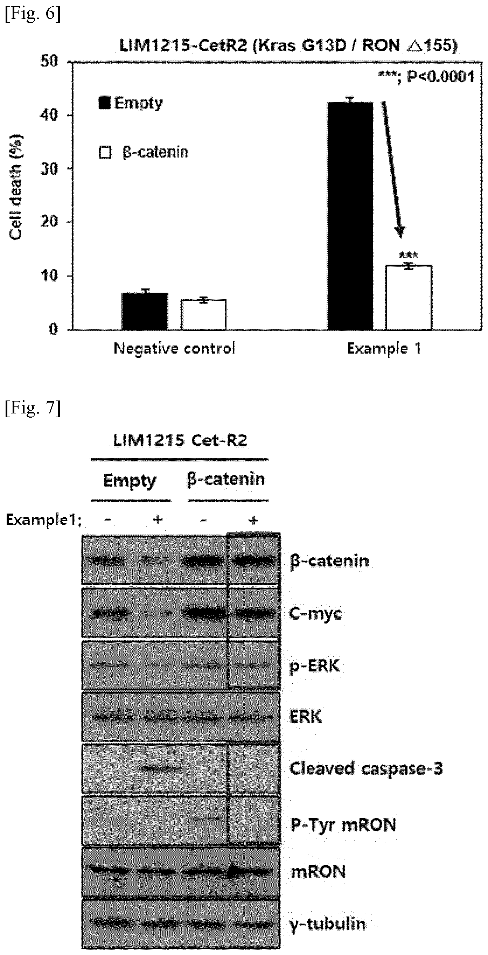

FIG. 6 shows results obtained by causing the colorectal cancer cell line LIM1215 having the KRAS G13D mutation and exhibiting resistance to cetuximab to overexpress β-catenin, and then treating the cell line with the compound of Example 1 to analyze the cell death rate.
FIG. 7 shows results obtained by identifying, with the western blotting method, changes in protein expression in the colorectal cancer cell line LIM1215 having the KRAS G13D mutation and exhibiting resistance to cetuximab when the cell line is caused to overexpress β-catenin, and then treated with the compound of Example 1.

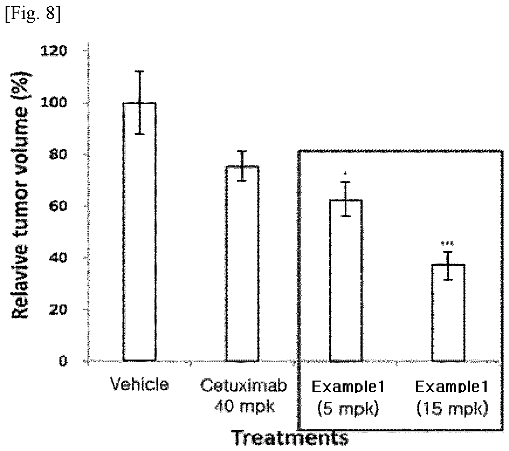

FIG. 8 shows results obtained by measuring a relative tumor volume when cetuximab and the compound of Example 1 (5 mg/kg (mpk), 15 mpk) are each administered to mouse models, transplanted with the colorectal cancer cell line LIM1215 having the KRAS G13D mutation which exhibits resistance to cetuximab.

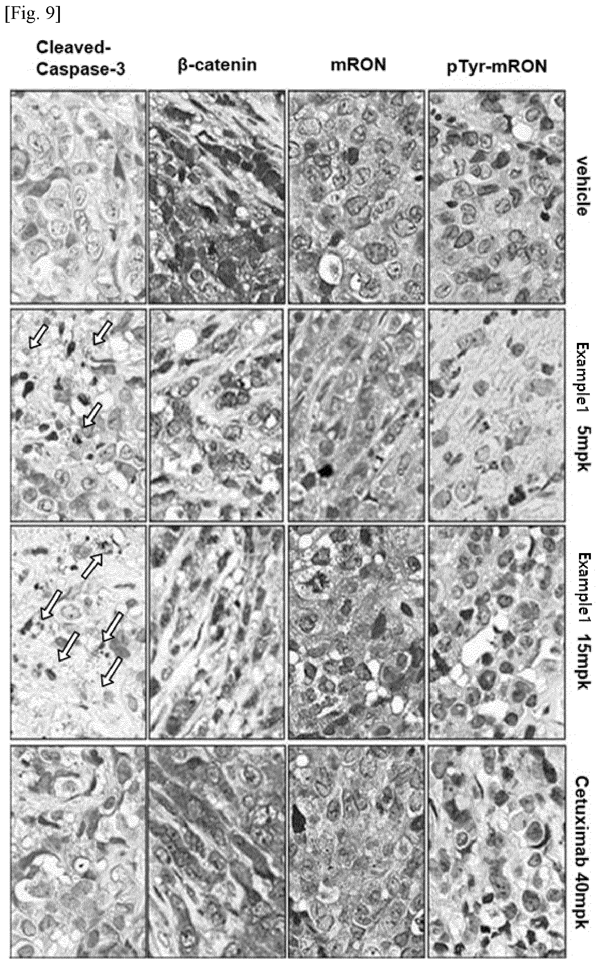

FIG. 9 shows results obtained by treating the colorectal cancer cell line LIM1215 having the KRAS G13D mutation and exhibiting resistance to cetuximab with the compound of Example 1 or cetuximab and analyzing, through immunohistochemical staining, whether changes in expression of pTyr-mRON, mRON, β-catenin, and cleaved caspase-3 have occurred in the cell line LIM1215.

### Best Mode for Carrying out the Invention

Hereinafter, the present invention will be described in detail.

In an aspect of the present invention, there is provided a pharmaceutical composition for preventing or treating cancer associated with a V-Ki-ras2 Kirsten rat sarcoma viral oncogene homolog (KRAS) mutation, the composition comprising, as an active ingredient, a compound represented by Formula 1 or Formula 2, or a pharmaceutically acceptable salt thereof.

The cancer, which the pharmaceutical composition of the present invention targets, exhibits resistance to an epidermal growth factor receptor (EGFR)-targeted therapeutic agent. In particular, in the present specification, the meaning of exhibiting resistance to an EGFR-targeted therapeutic agent may be interpreted as having secondary resistance to the drug.

As used herein, the term "KRAS" is also referred to as K-Ras and refers to a protein that constitutes part of RAS/MAPK, a cell signaling pathway that regulates cell growth, cell maturation, and cell death. Mutated versions of the KRAS are found in various types of solid cancers such as non-small cell lung cancer, colorectal cancer, and pancreatic cancer. In this case, the KRAS may have the amino acid sequence of SEQ ID NO: 1.

As used herein, the term "KRAS mutation" means that substitution, deletion, or insertion has occurred in some amino acids of KRAS. Specifically, the KRAS mutation may indicate that glycine, an amino acid residue, at position 13 in the amino acid sequence of SEQ ID NO: 1 is substituted with another amino acid. More specifically, the KRAS mutation may indicate that glycine, an amino acid residue, at position 13 in the amino acid sequence of SEQ ID NO: 1 is substituted with aspartic acid.

As used herein, the term "resistance" means that a drug in question does not have efficacy because the body does not respond sensitively to the drug.

As used herein, the term "EGFR-targeted therapeutic agent" refers to an anticancer drug targeting EGFR. Any EGFR-targeted therapeutic agent may be applied as long as the agent exhibits an anticancer effect. The EGFR-targeted therapeutic agent may be preferably cetuximab, gefitinib, erlotinib, or panitumumab, and most preferably cetuximab.

In the present invention, the resistance to an EGFR-targeted therapeutic agent may be associated with a recepteur d'origine nantais (RON) mutation. In addition, in the present invention, the RON mutation may be RONΔ155 in which exons 5, 6, and 11 are deleted. The cDNA of RONΔ 155 may have the amino acid sequence of SEQ ID NO: 2.

The RON refers to a protein receptor belonging to the c-MET family, and is a receptor for macrophage-stimulating protein (MSP), a serum protein, which is secreted by the liver and regulates the action of macrophages. The activity of RON plays an important role in the development, progression, and metastasis of tumors. In particular, overexpression or hyperactivity thereof in colorectal cancer and breast cancer has been reported to contribute to inducing tumor invasion and metastasis and inhibiting apoptosis.

In the present invention, the cancer may be selected from the group consisting of breast cancer, lung cancer, stomach cancer, prostate cancer, uterine cancer, ovarian cancer, kidney cancer, pancreatic cancer, liver cancer, colorectal cancer, skin cancer, head and neck cancer, and thyroid cancer.

Generally, in about 60% of colorectal cancers, cancer cells proliferate through a signaling pathway created by binding between epidermal growth factor receptor (EGFR) and epidermal growth factor (EGF). Specifically, when EGFR and EGF are bound to each other, KRAS gene is activated, causing karyomitosis of cancer cells. Cetuximab is an antibody targeting EGFR and inhibits activation of the KRAS gene by blocking the binding between EGFR and EGF. However, in a case where due to a mutation in the KRAS gene, the KRAS gene is activated without the binding between EGFR and EGF, cancer cells proliferate even when cetuximab is administered. In such a case where the KRAS gene is mutated, treatment is achieved using other targeted therapeutic agents such as crizotinib that is targeted to anaplastic lymphoma kinase (ALK). In particular, RON is overexpressed or excessively activated in colorectal cancer or breast cancer. Thus, in this respect, the compound or pharmaceutical composition of the present invention can be useful as an alternative anticancer agent in a case where cetuximab cannot be used due to a mutation in the KRAS gene.

In another aspect of the present invention, there is provided a use of the compound represented by Formula 1 or Formula 2 or a pharmaceutically acceptable salt thereof for preventing or treating cancer associated with the KRAS mutation.

In yet another aspect of the present invention, there is provided a use of the compound represented by Formula 1 or Formula 2 or a pharmaceutically acceptable salt thereof for preparation of a medicament for preventing or treating cancer associated with the KRAS mutation.

In still yet another aspect of the present invention, there is provided a method for preventing or treating cancer associated with the KRAS mutation, the method comprising administering the compound represented by Formula 1 or Formula 2 or a pharmaceutically acceptable salt thereof to a subject in need thereof.

In the present invention, the "prevention" refers to any act of inhibiting or delaying occurrence, spread, and recurrence of the disease; and the "treatment" refers to any act of ameliorating or beneficially altering symptoms of the disease by administration of the compound.

In addition, the term "subject" or "individual" refers to any animal, specifically a mammal, such as a monkey, a cow, a horse, a sheep, a pig, a chicken, a turkey, a quail, a cat, a dog, a mouse, a rat, a rabbit, and a guinea pig, including a human (patient), who has or is likely to develop the disease related to protein kinase activity. In addition, the subject in need thereof may mean a biological sample.

In addition, the "administration" means providing a predetermined substance to a subject in need thereof by any appropriate method, and administration of the compound of the present invention may be achieved via any common route as long as the route allows the substance to reach a target tissue.

In another aspect of the present invention, there is provided a method for treating a patient with cancer which exhibits resistance to an EGFR-targeted therapeutic agent, the method comprising: identifying whether the patient with cancer which exhibits resistance to an EGFR-targeted therapeutic agent has KRAS G13D mutation; and administering the compound represented by Formula 1 or Formula 2 or a pharmaceutically acceptable salt thereof to the patient with cancer having KRAS G13D mutation and exhibiting resistance to an EGFR-targeted therapeutic agent.

In yet another aspect of the present invention, there is provided a method for providing information on an anticancer therapeutic agent, the method comprising: identifying a KRAS mutation in an individual who exhibits resistance to an EGFR-targeted therapeutic agent; and providing information that the compound represented by Formula 1 or Formula 2 or a pharmaceutically acceptable salt thereof is suitable for anticancer therapy when a KRAS mutation, in which glycine, an amino acid residue, at position 13 in the amino acid sequence of SEQ ID NO: 1 is substituted with aspartic acid, is found.

The meaning of exhibiting resistance to the EGFR-targeted therapeutic agent is as described above. In addition, the EGFR-targeted therapeutic agent may be cetuximab, gefitinib, erlotinib, or panitumumab.

The compound of Formula 1 used in the present invention is represented as below: 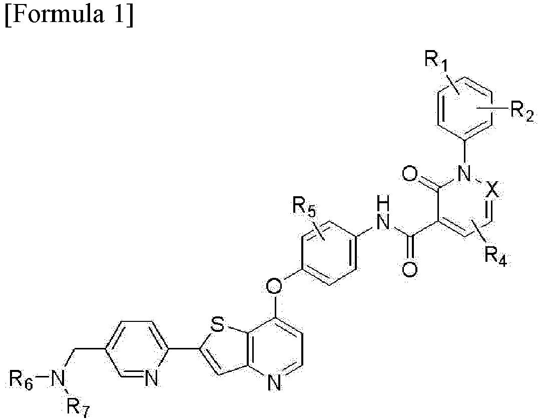
wherein, in Formula 1 above,
R₁ and R₂ are each independently H, halogen, C₁₋₁₀ alkoxy, or halo C₁₋₁₀ alkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ form a 4- to 10-membered heterocycle together with the N atom to which they are bonded, or R₆ is -C₂H₄-O-CH₃, and R₇ is H, methyl, or t-butoxycarbonyl; and
the heterocycle optionally further contains one or two heteroatoms selected from the group consisting of N, O, and S, in addition to the N atom to which R₆ and R₇ are bonded, and is unsubstituted or substituted with one or more substituents selected from among halogen and C₁₋₆ alkyl.

In addition, the C₁₋₁₀ alkyl may include C₁₋₆ alkyl, C₁₋₃ alkyl, C₃₋₁₀ alkyl, C₃₋₆ alkyl, C₆₋₁₀ alkyl, and the like. In addition, the C₁₋₁₀ alkoxy may include C₁₋₆ alkoxy, C₁₋₃ alkoxy, C₃₋₁₀ alkoxy, C₃₋₆ alkoxy, C₆₋₁₀ alkoxy, and the like. In addition, the 4-to 10-membered heterocycle may include 4- to 7-membered heterocycle, 4- to 6-membered heterocycle, 5- to 7-membered heterocycle, 5- or 6-membered heterocycle, and the like.

According to one embodiment, in Formula 1, R₁ and R₂ are each independently H, halogen, methoxy, or -CF₃, wherein the halogen may be F, Cl, Br, or I.

According to another embodiment, in Formula 1, R₃ and R₄ are each independently H, halogen, methyl, methoxy, or ethoxy, wherein the halogen may be F, Cl, Br, or I.

According to a further embodiment, in Formula 1, X is -C(-R₃)=; and R₃ and R₄ are each independently H, halogen, methyl, methoxy, or ethoxy, but not simultaneously H.

According to a still further embodiment, in Formula 1, X is -N=; and R₄ is halogen, methyl, methoxy, or ethoxy, wherein the halogen may be F, Cl, Br, or I.

According to a still further embodiment, in Formula 1, R₅ is H or halogen, wherein the halogen may be F, Cl, Br, or I.

According to a still further embodiment, in Formula 1, R₆ and R₇, taken together with the N atom to which they are bonded, form 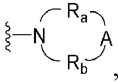 wherein Rₐ and R_{b} are each independently C₁₋₃ alkylene, A is -N(-R₉)- or -O-, and R₉ is C₁₋₆ alkyl. As specific examples, R₆ and R₇, together with the N atom to which they are bonded, may form a heterocycle group such as azetidinyl, diazetidinyl, pyrrolidinyl, pyrrolyl, imidazolidinyl, imidazolyl, pyrazolidinyl, pyrazolyl, oxazolidinyl, oxazolyl, isoxazolidinyl, isoxazolyl, thiazolidinyl, thiazolyl, isothiazolidinyl, isothiazolyl, piperidinyl, pyridinyl, piperazinyl, diazinyl, morpholino, thiomorpholino, azepanyl, and diazepanyl, which is optionally substituted with C₁₋₆ alkyl. Further, Rₐ and R_{b} may each independently be -CH₂-, -C₂H₄-, or -C₃H₆-. In addition, R₉ may be methyl, ethyl, n-propyl, i-propyl, n-butyl, i-butyl, t-butyl, n-pentyl, i-pentyl, t-pentyl, sec-pentyl, neopentyl, hexyl, and the like.

According to a still further embodiment, in Formula 1, R₁ and R₂ are each independently H, halogen, methoxy, or -CF₃; R₃ and R₄ are each independently H, halogen, methyl, methoxy, or ethoxy; R₅ is H or halogen; and R₆ is -C₂H₄-O-CH₃ and R₇ is H, methyl, or t-butoxycarbonyl, or R₆ and R₇ may be bonded together to form a morpholino or methylpiperazinyl group. Said halogen may be F, Cl, Br, or I.

According to one embodiment, the compound of Formula 1 may be represented by Formula 1a below. 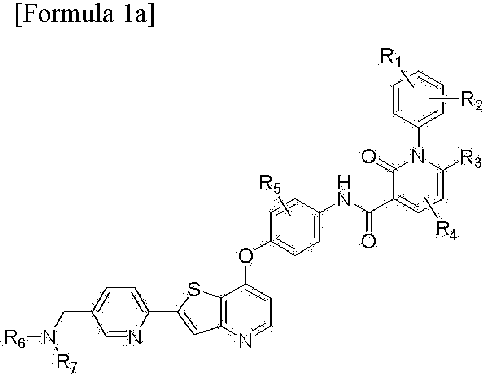 wherein R₁ to R₇ are the same as defined in the above Formula 1.

Specifically, in the above Formula 1a, R₁ and R₂ may each independently be H, halogen, or -CF₃. Further, R₃ and R₄ may each independently be H, halogen, methyl, methoxy, or ethoxy, but are not simultaneously H. In addition, R₅ may be H or halogen.

More specifically, in the above Formula 1a, R₁ and R₂ may each independently be H, halogen, or -CF₃; R₃ and R₄ may each independently be H, halogen, methyl, methoxy, or ethoxy; R₅ may be H or halogen; R₆ may be -C₂H₄-O-CH₃; and R₇ may be H, methyl, or t-butoxycarbonyl.

According to another embodiment, the compound of Formula 1 may be represented by Formula 1b below. 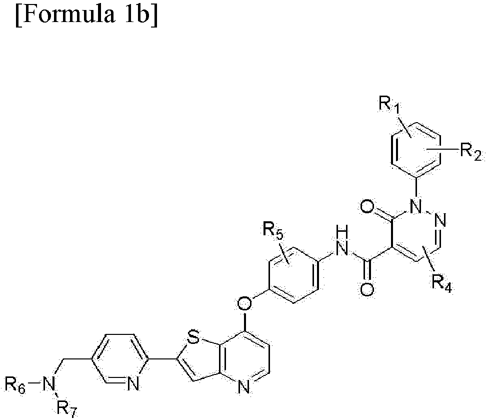 wherein R₁ to R₇ are the same as defined in the above Formula 1.

Specifically, in the above Formula 1b, R₁ and R₂ may each independently be H, halogen, or -CF₃. Further, R₄ may be halogen, methyl, methoxy, or ethoxy. In addition, R₅ may be H or halogen.

More specifically, in the above Formula 1b, R₁ and R₂ may each independently be H, halogen, or -CF₃; R₄ may be halogen, methyl, methoxy, or ethoxy; R₅ may be H or halogen; R₆ may be -C₂H₄-O-CH₃; and R₇ may be H, methyl, or t-butoxycarbonyl.

According to a further embodiment, the compound of Formula 1 may be represented by Formula 1c below. 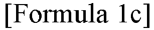 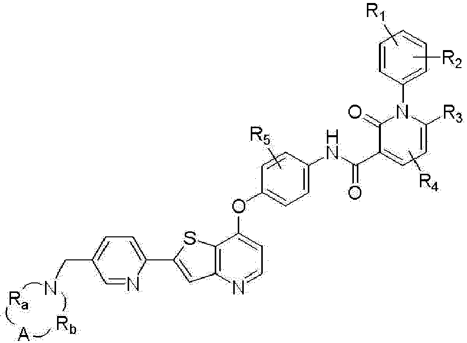 wherein R₁ to R₅ are the same as defined in the above Formula 1; Rₐ and R_{b} may each independently be C₁₋₃ alkylene; A may be -N(-R₉)-, or -O-; and R₉ may be C₁₋₆ alkyl.

Specifically, in the above Formula 1c, R₁ and R₂ may each independently be H, halogen, or -CF₃. Further, R₃ and R₄ may each independently be H, halogen, methyl, methoxy, or ethoxy, but are not simultaneously H. In addition, R₅ may be H or halogen. Further, Rₐ and R_{b} may form a morpholino or methylpiperazinyl group together with N and A to which they are bonded.

According to a still further embodiment, the compound of Formula 1 may be represented by Formula 1d below. 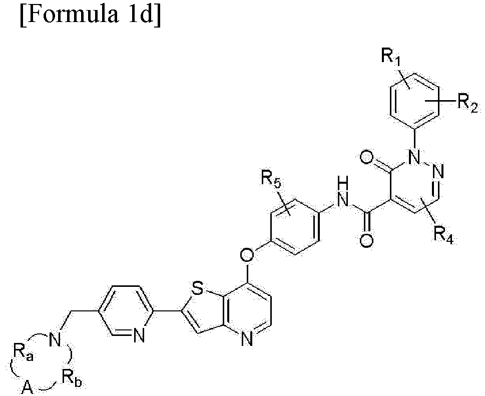 wherein R₁ to R₅ are the same as defined in the above Formula 1; Rₐ and R_{b} may each independently be C₁₋₃ alkylene; A may be -N(-R₉)- or -O-; and R₉ may be C₁₋₆ alkyl.

Specifically, in the above Formula 1d, R₁ and R₂ may each independently be H, halogen, or -CF₃. Further, R₄ may be halogen, methyl, methoxy, or ethoxy. In addition, R₅ may be H or halogen. Further, Rₐ and R_{b} may form a morpholino or methylpiperazinyl group together with N and A to which they are bonded.

Specific examples of the compound of Formula 1 are listed below:
1) 4-Ethoxy-N-(3-fluoro-4-{ [2-(5-{ [(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide;
2) N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3 -carboxamide;
3) N-(3-fluoro-4-{[2-(5-{[(2-methoxyethyl)amino]methyl}pyridin-2-yl)thieno[3,2-b]pyridin-7-yl]oxy}phenyl)-4-methoxy-2-oxo-1-phenyl-1,2-dihydropyridine-3 -carboxamide;
4) N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3 -carboxamide;
5) N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide;
6) t-Butyl {[6-(7-{4-[4-ethoxy-1-(4-fluorophenyl)-2-oxo-1,2-dihydropyridine-3-carboxamido]-2-fluorophenoxy}thieno[3,2-b]pyridin-2-yl)pyridin-3-yl]methyl}(2-methoxyethyl)carbamate;
7) 4-ethoxy-N-(3-fhioro-4-{[2-(5-{[(2-methoxyethyl)amino]methyl}pyridin-2-yl)thieno[3,2-b]pyridin-7-yl]oxy}phenyl)-1-(4-fluorophenyl)-2-oxo-1,2-dihydropyridine-3 -carboxamide;
8) 1-(4-chlorophenyl)-4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1,2-dihydropyridine-3 -carboxamide;
9) N-(3-chloro-4-{ [2-(5-{ [(2-methoxyethyl)amino]methyl}pyridin-2-yl)thieno[3,2-b]pyridin-7-yl]oxy}phenyl)-1-(4-fluorophenyl)-4-methoxy-2-oxo-1,2-dihydropyridine-3 -carboxamide;
10) N-(2-chloro-4-1[-2-(5-1[(2-methoxyethyl)amino]methyllpyridin-2-yl)thieno[3,2-b]pyridin-7-yl]oxy}phenyl)-1-(4-fluorophenyl)-4-methoxy-2-oxo-1,2-dihydropyridine-3 -carboxamide;
11) 1-(4-fluorophenyl)-4-methoxy-N-(4-{[2-(5-{[(2-methoxyethyl)amino]methyl}pyridm-2-yl)thieno[3,2-b]pyridm-7-yl)oxy)phenyl)-2-oxo-1,2-dihydropyridine-3 -carboxamide;
12) 4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-(4-(trifluoromethyl)phenyl)-1,2-dihydropyridine-3 -carboxamide;
13) 1-(4-chlorophenyl)-N-(3-fluoro-4-{[2-(5-{[(2-methoxyethyl)amino]methyl}pyridin-2-yl)thieno[3,2-b]pyridin-7-yl]oxy}phenyl)-4-methoxy-2-oxo-1,2-dihydropyridine-3-carboxamide;
14) 4-ethoxy-N-(3-fluoro-4-{[2-(5-{[(2-methoxyethyl)amino]methyl}pyridm-2-yl)thieno[3,2-b]pyridin-7-yl]oxy}phenyl)-1-(3-fluorophenyl)-2-oxo-1,2-dihydropyridine-3 -carboxamide;
15) 4-ethoxy-N-(3-fluoro-4-{[2-(5-{[(2-methoxyethyl)amino]methyl}pyridm-2-yl)thieno[3,2-b]pyridin-7-yl]oxy}phenyl)-1-(4-methoxyphenyl)-2-oxo-1,2-dihydropyridine-3 -carboxamide;
16) 4-ethoxy-N-(3-fluoro-4-{[2-(5-{[(2-methoxyethyl)amino]methyl}pyridm-2-yl)thieno[3,2-b]pyridin-7-yl]oxy}phenyl)-1-(3-methoxyphenyl)-2-oxo-1,2-dihydropyridine-3 -carboxamide;
17) N-(3-fluoro-4-{ [2-(5-{ [(2-methoxyethyl)ammo]methyl}pyridm-2-yl)thieno[3,2-b]pyridin-7-yl]oxy}phenyl)-2-(4-fluorophenyl)-5-methyl-3-oxo-2,3-dihydropyridazine-4-carboxamide;
18) N-(3-fluoro-4-{[2-(5-{[(2-methoxyethyl)amino]methyl}pyridin-2-yl)thieno[3,2-b]pyridin-7-yl]oxy}phenyl)-5-methyl-3-oxo-2-phenyl-2,3-dihydropyridazine-4-carboxamide;
19) N-(3-fluoro-4-{[2-(5-{ [(2-methoxyethyl)amino]methyl}pyridm-2-yl)thieno[3,2-b]pyridin-7-yl]oxy}phenyl)-3-oxo-2-phenyl-2,3-dihydropyridazine-4-carboxamide;
20) N-(3-fluoro-4-[{2-(5-[{(2-methoxyethyl)amino}methyl]pyridm-2-yl)thieno[3,2-b]pyridin-7-yl}oxy]phenyl)-2-(4-fluorophenyl)-3-oxo-2,3-dihydropyridazine-4-carboxamide;
21) N-(3-fluoro-4-{[2-(5-{[(2-methoxyethyl)amino]methyl}pyridm-2-yl)thieno[3,2-b]pyridin-7-yl]oxy}phenyl)-6-methyl-2-oxo-1-phenyl-1,2-dihydropyridine-3-carboxamide;
22) N-(3-fluoro-4-{ [2-(5-{ [(2-methoxyethyl)ammo]methyl}pyridm-2-yl)thieno[3,2-b]pyridin-7-yl]oxy}phenyl)-1-(4-fluorophenyl)-6-methyl-2-oxo-1,2-dihydropyridine-3-carboxamide;
23) 5-Bromo-N-(3-fluoro-4-{[2-(5-{[(2-methoxyethyl)amino]methyl}pyridm-2-yl)thieno[3,2-b]pyridin-7-yl]oxy}phenyl)-1-(4-fluorophenyl)-2-oxo-1,2-dihydropyridine-3-carboxamide;
24) 5-Chloro-N-(3-fluoro-4-{ [2-(5-{ [(2-methoxyethyl)ammo]methyl}pyridm-2-yl)thieno[3,2-b]pyridin-7-yl]oxy}phenyl)-1-(4-fluorophenyl)-2-oxo-1,2-dihydropyridine-3-carboxamide;
25) N-(3-fluoro-4-{[2-(5-{[(2-methoxyethyl)amino]methyl}pyridin-2-yl)thieno[3,2-b]pyridin-7-yl]oxy}phenyl)-1-(4-fluorophenyl)-4-methyl-2-oxo-1,2-dihydropyridine-3-carboxamide;
26) N-(2-chloro-4-{[2-(5-{[(2-methoxyethyl)amino]methyl}pyridin-2-yl)thieno[3,2-b]pyridin-7-yl]oxy}phenyl)-4-ethoxy-1-(4-fluorophenyl)-2-oxo-1,2-dihydropyridine-3-carboxamide;
27) N-(3-fluoro-4-([2-(5-{[(2-methoxyethyl)amino)methyl]pyridm-2-yl}thieno[3,2-b]pyridin-7-yl)oxy]phenyl}-1-(4-fluorophenyl)-5,6-dimethyl-2-oxo-1,2-dihydropyridine-3-carboxamide;
28) N-(3-fluoro-4-{[-2-(5-{[(2-methoxyethyl)amino]methyl}pyridin-2-yl)thieno[3,2-b]pyridin-7-yl]oxylphenyl)-4-methyl-2-oxo-1-phenyl-1,2-dihydropyridine-3-carboxamide;
29) N-(3-fluoro-4-{[-2-(5-{[(2-methoxyethyl)amino]methyl}pyridin-2-yl)thieno[3,2-b]pyridin-7-yl]oxy}phenyl)-1-(4-fluorophenyl)-5-methyl-2-oxo-1,2-dihydropyridine-3 -carboxamide;
30) 4-Ethoxy-N-(3-fluoro-4-{[2-(5-{[(2-methoxyethyl)(methyl)amino]methyl}pyridin-2-yl)thieno[3,2-b]pyridin-7-yl]oxy}phenyl)-1-(4-fluorophenyl)-2-oxo-1,2-dihydropyridine-3-carboxamide;
31) 4-ethoxy-N-[3-fhioro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3 -carboxamide;
32) 4-ethoxy-N-[3-fhioro-4-({2-[5-(morpholinomethyl)pyridin-2-yl]thieno[3,2-b]pyridin-7-yl}oxy)phenyl]-2-oxo-1-phenyl-1,2-dihydropyridine-3-carboxamide;
33) 4-ethoxy-N-{3-fluoro-4-[(2-{5-[(4-methylpiperazin-1-yl)methyl]pyridin-2-yl}thieno[3,2-b]pyridin-7-yl)oxy]phenyl}-1-(4-fluorophenyl)-2-oxo-1,2-dihydropyridine-3 -carboxamide;
34) 4-ethoxy-N-13-fluoro-4-[(2-15-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide;
35) 1-(4-chlorophenyl)-4-ethoxy-N-[3-fluoro-4-({2-[5-(morpholinomethyl)pyridin-2-yl]thieno[3,2-b]pyridin-7-yl}oxy)phenyl]-2-oxo-1,2-dihydropyridine-3 -carboxamide;
36) N-[3-chloro-4-({2-[5-(morpholinomethyl)pyridin-2-yl]thieno[3,2-b]pyridin-7-yl}oxy)phenyl]-4-ethoxy-1-(4-fluorophenyl)-2-oxo-1,2-dihydropyridine-3-carboxamide; and
37) N-[2-chloro-4-({2-[5-(morpholinomethyl)pyridin-2-yl]thieno[3,2-b]pyridin-7-yl}oxy)phenyl]-4-ethoxy-1-(4-fluorophenyl)-2-oxo-1,2-dihydropyridine-3-carboxamide.

Preferably, the compounds of Formula 1 may be selected from the group consisting of:
4-ethoxy-N-[3-fhioro-4-({2-[5-(morpholinomethyl)pyridin-2-yl]thieno[3,2-b]pyridin-7-yl} oxy)phenyl] -1-(4-fluorophenyl)-2-oxo-1,2-dihydropyridine-3 - carboxamide;
4-Ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide; and
4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

The compound of Formula 1 above used in the composition according to the present invention may be prepared by a method disclosed in Korean Patent Laid-open Publication No. 2019-0106802, and by other known methods and/or various methods based on the technology in the field of organic synthesis. Based on the above methods, various derivatives may be synthesized using an appropriate synthesis method according to the type of substituent.

The compound of Formula 2 used in the present invention is represented as below: 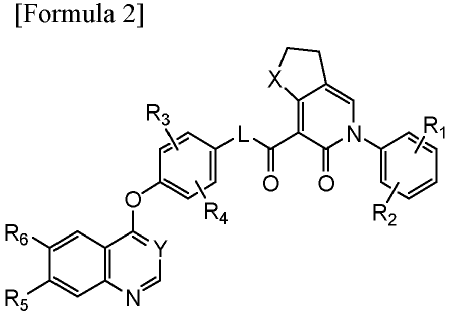
in the formula 2,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
Y is -N= or -CH=,
R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, or C₁₋₆ alkylamino-C₁₋₆ alkoxy,
wherein R₅ and R₆ are each independently optionally substituted with 3- to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contain 1 to 4 heteroatoms selected from the group consisting of N, O, and S.
In addition, the C₁₋₆ alkyl may include C₁₋₃ alkyl, C₃₋₆ alkyl, and the like. In addition, the C₁₋₆ alkoxy may include C₁₋₃ alkoxy, C₃₋₆ alkoxy, and the like.

According to an embodiment, in Formula 2, R₁ to R₄ may each independently be hydrogen, C₁₋₄ haloalkyl, or halogen. Here, the halogen may be F, Cl, Br, or I. Specifically, R₁ may be hydrogen, trifluoromethyl, or fluoro; R₂ may be hydrogen; R₃ may be fluoro; and R₄ may be hydrogen.

According to another embodiment, in Formula 2, X may be O or -CH(-Rx)-and Rx may be hydrogen or C₁₋₆ alkyl.

According to still yet another embodiment, in Formula 2, R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9- membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

Specifically, the heteroaryl may be pyridinyl, imidazolyl, or pyrazolyl; the heterocycloalkyl may be azetidinyl, pyrrolidinyl, tetrahydropyranyl, morpholino, morpholinyl, dioxidothiomorpholino, piperazinyl, piperidinyl, or oxetanyl; and the cycloalkyl may be cyclobutyl, cyclopentyl, or cyclohexyl.

In addition, in a case where the heteroaryl or the heterocycloalkyl contains one or more N atoms, substitution may be made at any one of the N atomic positions. However, there is no particular limitation thereon.

According to still yet another embodiment, in Formula 2, R₁ and R₂ are hydrogen, C₁₋₄ haloalkyl, or halogen; R₃ and R₄ are hydrogen or halogen; X is O or - CH(-Rx)- and Rx is hydrogen or C₁₋₄ alkyl; A is quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine, or indazole; and R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, C₁₋₆ alkylamino-C₁₋₆ alkoxy, or 5- to 9-membered heteroaryl, wherein R₅ and R₆ may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3-to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 2, R₅ and R₆ are each independently hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, C₁₋₆ alkylamino-C1- 6 alkoxy, C₆₋₁₀ aryl, C₆₋₁₀ aryl-C₁₋₄ alkyl, or 5- to 9-membered heteroaryl, wherein the amino, the alkyl, the alkoxy, the aryl, and the heteroaryl may each independently be optionally substituted with C₁₋₆ alkyl; C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl; 3- to 9-membered cycloalkyl; or 3- to 9-membered heterocycloalkyl; the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heterocyclic ring, the heteroaryl and the heterocycloalkyl may each independently contain one or more heteroatoms selected from the group consisting of N, O, and S.

According to still yet another embodiment, in Formula 2, R₅ and R₆ may not be, at the same time, C₆₋₁₀ aryl, C₆₋₁₀ aryl-C₁₋₄ alkyl, or 5- to 9-membered heteroaryl. According to still yet another embodiment, in Formula 2, R₅ and R₆ may not be substituted, at the same time, with C₁₋₆ alkyl or C₁₋₆ alkylamino-C₁₋₆ alkyl, substituted with any one of 3- to 9-membered cycloalkyl and 3- to 9-membered heterocycloalkyl; 3- to 9-membered cycloalkyl; or 3- to 9-membered heterocycloalkyl. As a specific example, in a case where R₅ contains a ring such as aryl or heteroaryl, R₆ may not contain this ring at the same time. In addition, in a case where R₅ is substituted with a group containing a ring such as cycloalkyl or heterocycloalkyl, R₆ may not be substituted, at the same time, with a group containing this ring.

As a more specific example, R₅ may be C₆₋₁₀ aryl, C₆₋₁₀ aryl-C₁₋₄ alkyl, or 5-to 9-membered heteroaryl; and R₆ may be hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylaminocarbonyl, C₁₋₆ alkylcarbonylamino, C₁₋₆ alkylamino, or C₁₋₆ alkylamino-C₁₋₆ alkoxy. Here, R₅ may be optionally substituted with C₁₋₆ alkyl; or C₁₋₆ alkyl or C₁₋₆ alkylamino-C₁₋₆ alkyl, substituted with any one of C₁₋₆ alkoxy-C₁₋₆ alkylamino, 3- to 9-membered cycloalkyl, and 3- to 9-membered heterocycloalkyl. In addition, R₆ may be optionally substituted with 3- to 9-membered cycloalkyl or 3- to 9-membered heterocycloalkyl. Here, the cycloalkyl or the heterocycloalkyl may optionally have one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl; and the heteroaryl and the heterocycloalkyl may each independently contain one or more heteroatoms selected from the group consisting of N, O, and S.

According to still yet another embodiment, in Formula 2, R₅ and R₆ are each independently hydrogen, amino, halogen, hydroxy, C₁₋₆ alkoxy, aminocarbonyl, C₁₋₆ alkylaminocarbonyl, diC₁₋₆ alkylaminocarbonyl, C₁₋₆ alkylcarbonylamino, cyano, C₁₋₄ haloalkyl, C₁₋₆ alkyl, 5- or 9-membered heteroaryl, Ax-(CH₂)ₐ-L1-(CH₂)_{b}-L2-, or Ax-(CH₂)ₐ-L1-(CH₂)_{b}-L2-pyridinyl; Ax is C₃₋₆ cycloalkyl or 3-6 membered heterocycloalkyl; L1 and L2 are each independently a single bond, -O-, -NH-, - C(=O)-NH-, or -NH-C(=O)-; and a and b are each independently an integer of 0 to 3, provided that in a case where b is 0, L2 is a single bond, wherein the cycloalkyl, the heteroaryl, and the heterocycloalkyl may each independently optionally have one or two substituents selected from the group consisting of halogen, oxo, cyano, hydroxy, hydroxymethyl, C₁₋₆ alkyl, methoxy, methoxymethyl, dimethylamino, and methoxyethylaminomethyl; the heteroaryl and the heterocycloalkyl may each independently contain one or more heteroatoms selected from the group consisting of N, O, and S; and the heteroaryl and the heterocycloalkyl may each independently contain one or more heteroatoms selected from the group consisting of N, O, and S.

Specific examples of the compound represented by Formula 2 are as follows:
40) N-(4-((6,7-dimethoxyquinolin-4-yl)oxy)-3-fluorophenyl)-5-(4-fluorophenyl)-6-oxo-2,3,5,6-tetrahydrofuro[3,2-c]pyridine-7-carboxamide;
41) N-(4-((6,7-dimethoxyquinolin-4-yl)oxy)-3 -fluorophenyl)-6-oxo-5-phenyl-2,3,5,6-tetrahydrofuro[3,2-c]pyridine-7-carboxamide;
42) N-(4-((6,7-dimethoxyquinolin-4-yl)oxy)-3-fluorophenyl)-6-oxo-5-(4-(trifluoromethyl)phenyl)-2,3,5,6-tetrahydrofuro [3,2-c]pyridine-7-carboxamide;
43) N-(4-((6,7-dimethoxyquinolin-4-yl)oxy)-3-fluorophenyl)-6-oxo-5-(3-fluorophenyl)-2,3,5,6-tetrahydrofuro[3,2-c]pyridine-7-carboxamide;
44) N-(3-fluoro-4-((6-methoxyquinolin-4-yl)oxy)phenyl)-5-(4-fluorophenyl)-6-oxo-2,3,5,6-tetrahydrofuro[3,2-c]pyridine-7-carboxamide;
45) N-(3-fluoro-4-((7-methoxyquinolin-4-yl)oxy)phenyl)-5-(4-fluorophenyl)-6-oxo-2,3,5,6-tetrahydrofuro[3,2-c]pyridine-7-carboxamide;
46) N-(4-((6,7-dimethoxyquinazolin-4-yl)oxy)-3-fluorophenyl)-5-(4-fluorophenyl)-6-oxo-2,3,5,6-tetrahydrofuro[3,2-c]pyridine-7-carboxamide;
47) N-(4-((6-carbamoyl-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
48) N-(3-fluoro-4-((7-methoxy-6-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
49) N-(4-((6-(dimethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
50) N-(3-fluoro-4-((7-methoxy-6-((2-morpholinoethyl)carbamoyl)quinolin-4-yl)oxy)phenyl)-5-(4-fluorophenyl)-6-oxo-2,3,5,6-tetrahydrofuro[3,2-c]pyridine-7-carboxamide;
51) N-(4-((6-(ethylcarbamoyl)-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
52) N-(4-((6-acetamido-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
53) N-(3-fluoro-4-((7-methoxy-6-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
76) N-(3-fluoro-4-((6-methoxy-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
77) N-(3-fluoro-4-((6-methoxy-7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
78) N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
79) N-(3-fluoro-4-((6-methoxy-7-(2-morpholinoethoxy)quinolin-4-yl)oxy)phenyl)-6-oxo-5-phenyl-2,3,5,6-tetrahydrofuro[3,2-c]pyridine-7-carboxamide;
80) N-(3-fluoro-4-((6-methoxy-7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
81) N-(3-fluoro-4-((7-(3-(3-hydroxyazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
82) N-(3-fluoro-4-((7-(3-(3-hydroxy-3-methylazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
83) N-(3-fluoro-4-((7-(3-(3-(hydroxymethyl)azetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
84) N-(3-fluoro-4-((6-methoxy-7-(3-(3-methoxyazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
85) N-(3-fluoro-4-((6-methoxy-7-(3-(3-methoxy-3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
86) N-(3-fluoro-4-((7-(3-(3-fluoroazetidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
87) N-(4-((7-(3-(3,3-difluoroazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
88) N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
89) N-(3-fluoro-4-((6-methoxy-7-(3-(3-methylazetidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
90) N-(3-fluoro-4-((7-(3-(3-hydroxypyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
91) N-(3-fluoro-4-((7-(3-(3-hydroxy-3-methylpyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
92) N-(3-fluoro-4-((6-methoxy-7-(3-(3-methoxypyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
93) N-(3-fluoro-4-((6-methoxy-7-(3-(pyrrolidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
94) N-(3-fluoro-4-((7-(3-(3-fluoropyrrolidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
95) N-(4-((7-(3-(3,3-difluoropyrrolidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
96) N-(3-fluoro-4-((7-(3-(4-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
97) N-(3-fluoro-4-((7-(3-(3-hydroxypiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
98) N-(3-fluoro-4-((7-(3-(4-hydroxy-4-methylpiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
99) N-(3-fluoro-4-((6-methoxy-7-(3-(4-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
100) N-(3-fluoro-4-((6-methoxy-7-(3-(3-methoxypiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
101) N-(3-fluoro-4-((6-methoxy-7-(3-(4-oxopiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
102) N-(4-((7-(3-(1,1-dioxidothiomorpholino)propoxy)-6-methoxyqumolm-4-yl)oxy)-3-fluorophenyl)-5-(4-fluorophenyl)-6-oxo-2,3,5,6-tetrahydrofuro[3,2-c]pyridine-7-carboxamide;
103) N-(3-fluoro-4-((6-methoxy-7-(3-(piperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
104) N-(3-fluoro-4-((7-(3-(4-fluoropiperidin-1-yl)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
105) N-(4-((7-(3-(4,4-difluoropiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
106) N-(3-fluoro-4-((6-methoxy-7-(3-(4-methylpiperidin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
107) N-(4-((7-(3-(4,4-dimethylpiperidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
108) N-(3 -fluoro-4-((7-(3 -((3 -hydroxycyclobutyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
109) N-(3-fluoro-4-((6-methoxy-7-(3-((3-methoxycyclobutyl)amino)propoxy)quinolin-4-yl)oxy)phenyl)-5-(4-fluorophenyl)-6-oxo-2,3,5,6-tetrahydrofuro[3,2-c]pyridine-7-carboxamide;
110) N-(3-fluoro-4-((6-methoxy-7-(3-(oxetan-3-ylamino)propoxy)quinolin-4-yl)oxy)phenyl)-5-(4-fluorophenyl)-6-oxo-2,3,5,6-tetrahydrofuro[3,2-c]pyridine-7-carboxamide;
111) N-(3-fluoro-4-((6-methoxy-7-(3-((oxetan-3-ylmethyl)amino)propoxy)quinolin-4-yl)oxy)phenyl)-5-(4-fluorophenyl)-6-oxo-2,3,5,6-tetrahydrofuro[3,2-c]pyridine-7-carboxamide;
112) N-(3-fluoro-4-((7-(3-((3-hydroxycyclopentyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
113) N-(3-fluoro-4-((7-(3-((3-hydroxycyclohexyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
114) N-(3-fluoro-4-((7-(3-(((3-hydroxycyclohexyl)methyl)amino)propoxy)-6-methoxyquinolin-4-yl)oxy)phenyl)-5-(4-fluorophenyl)-6-oxo-2,3,5,6-tetrahydrofuro[3,2-c]pyridine-7-carboxamide;
115) N-(3-fluoro-4-((6-methoxy-7-(3-((tetrahydro-2H-pyran-4-yl)amino)propoxy)quinolin-4-yl)oxy)phenyl)-5-(4-fluorophenyl)-6-oxo-2,3,5,6-tetrahydrofuro[3,2-c]pyridine-7-carboxamide;
116) N-(3-fluoro-4-((6-methoxy-7-(3-(((tetrahydro-2H-pyran-4-yl)methyl)amino)propoxy)quinolin-4-yl)oxy)phenyl)-5-(4-fluorophenyl)-6-oxo-2,3,5,6-tetrahydrofuro[3,2-c]pyridine-7-carboxamide;
117) N-(3-fluoro-4-((7-(2-(3-hydroxyazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
118) N-(3-fluoro-4-((7-(2-(3-hydroxy-3-methylazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
119) N-(3-fluoro-4-((7-(2-(3-(hydroxymethyl)azetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
120) N-(3-fluoro-4-((6-methoxy-7-(2-(3-methoxyazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
121) N-(3-fluoro-4-((6-methoxy-7-(2-(3-methoxy-3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
122) N-(3-fluoro-4-((6-methoxy-7-(2-(3-(methoxymethyl)azetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
123) N-(3-fluoro-4-((7-(2-(3-fluoroazetidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
124) N-(4-((7-(2-(3,3-difluoroazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
125) N-(4-((7-(2-(3-ethynylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
126) N-(3-fluoro-4-((6-methoxy-7-(2-(3-methylazetidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
127) N-(4-((7-(2-(3,3-dimethylazetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
128) N-(4-((7-(2-(3-(dimethylamino)azetidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
129) N-(3-fluoro-4-((7-(2-(3-hydroxypyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
130) N-(3-fluoro-4-((7-(2-(3-hydroxy-3-methylpyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
131) N-(3-fluoro-4-((6-methoxy-7-(2-(3-methoxypyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
132) N-(3-fluoro-4-((6-methoxy-7-(2-(pyrrolidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
133) N-(3-fluoro-4-((7-(2-(3-fluoropyrrolidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
134) N-(4-((7-(2-(3,3-difluoropyrrolidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
135) N-(3-fluoro-4-((7-(2-(4-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
136) N-(3-fluoro-4-((7-(2-(3-hydroxypiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
13 7) N-(3-fluoro-4-((7-(2-(4-hydroxy-4-methylpiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
138) N-(3-fluoro-4-((6-methoxy-7-(2-(4-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
139) N-(3-fluoro-4-((6-methoxy-7-(2-(3-methoxypiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
140) N-(3-fluoro-4-((6-methoxy-7-(2-(4-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
141) N-(3-fluoro-4-((6-methoxy-7-(2-(3-oxopiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
142) N-(4-((7-(2-(1,1-dioxidothiomorpholino)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
143) N-(3-fluoro-4-((6-methoxy-7-(2-(piperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
144) N-(3-fluoro-4-((7-(2-(4-fluoropiperidin-1-yl)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
145) N-(4-((7-(2-(4,4-difluoropiperidin-1-yl)ethoxy)-6-methoxyqumolm-4-yl)oxy)-3-fluorophenyl)-5-(4-fluorophenyl)-6-oxo-2,3,5,6-tetrahydrofuro[3,2-c]pyridine-7-carboxamide;
146) N-(3-fluoro-4-((6-methoxy-7-(2-(4-methylpiperidin-1-yl)ethoxy)quinolin-4-yl)oxy)phenyl)-5-(4-fluorophenyl)-6-oxo-2,3,5,6-tetrahydrofuro[3,2-c]pyridine-7-carboxamide;
147) N-(4-((7-(2-(4,4-dimethylpiperidin-1-yl)ethoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
148) N-(3-fluoro-4-((7-(2-((3-hydroxycyclobutyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
149) N-(3-fluoro-4-((6-methoxy-7-(2-((3-methoxycyclobutyl)amino)ethoxy)qumolm-4-yl)oxy)phenyl)-5-(4-fluorophenyl)-6-oxo-2,3,5,6-tetrahydrofuro[3,2-c]pyridine-7-carboxamide;
150) N-(3-fluoro-4-((6-methoxy-7-(2-(oxetan-3-ylamino)ethoxy)quinolin-4-yl)oxy)phenyl)-5-(4-fluorophenyl)-6-oxo-2,3,5,6-tetrahydrofuro[3,2-c]pyridine-7-carboxamide;
151) N-(3-fluoro-4-((6-methoxy-7-(2-((oxetan-3-ylmethyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
152) N-(3-fluoro-4-((7-(2-((4-hydroxycyclohexyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
153) N-(3-fluoro-4-((7-(2-((3-hydroxycyclohexyl)amino)ethoxy)-6-methoxyquinolmin4-yl)oxy)phenyl)-5-(4-fluorophenyl)-6-oxo-2,3,5,6-tetrahydrofuro[3,2-c]pyridine-7-carboxamide;
154) N-(3-fluoro-4-((7-(2-(((3-hydroxycyclohexyl)methyl)amino)ethoxy)-6-methoxyquinolin-4-yl)oxy)phenyl)-5-(4-fluorophenyl)-6-oxo-2,3,5,6-tetrahydrofuro[3,2-c]pyridine-7-carboxamide;
155) N-(3-fluoro-4-((6-methoxy-7-(2-((tetrahydro-2H-pyran-4-yl)amino)ethoxy)quinolin-4-yl)oxy)phenyl)-5-(4-fluorophenyl)-6-oxo-2,3,5,6-tetrahydrofuro[3,2-c]pyridine-7-carboxamide;
156) N-(3-fluoro-4-((6-methoxy-7-(2-((4-methoxycyclohexyl)amino)ethoxy)quinolin-4-yl)oxy)phenyl)-5-(4-fluorophenyl)-6-oxo-2,3,5,6-tetrahydrofuro[3,2-c]pyridine-7-carboxamide;
157) N-(3-fluoro-4-((7-(2-morpholinylethoxy)quinolin-4-yl)oxy)phenyl)-5-(4-fluorophenyl)-6-oxo-2,3,5,6-tetrahydrofuro[3,2-c]pyridine-7-carboxamide;
158) N-(3-fluoro-4-((7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
159) N-(3-fluoro-4-((7-(3-morpholinopropoxy)quinolin-4-yl)oxy)phenyl)-2-(4-fluorophenyl)-3-oxo-3,5,6,7-tetrahydro-2H-cyclopenta[c]pyridine-4-carboxamide;
160) N-(3-fluoro-4-((7-(3-(4-methylpiperazin-1-yl)propoxy)quinolin-4-yl)oxy)phenyl)-5-(4-fluorophenyl)-6-oxo-2,3,5,6-tetrahydrofuro[3,2-c]pyridine-7-carboxamide;
161) 7-(2-(3-fluoro-4-((7-(3-(piperidin-1-yl)propoxy)quinolin-4-yl)oxy)phenyl)acetyl)-5-(4-fluorophenyl)-3,5-dihydrofuro[3,2-c]pyridin-6(2H)-one;
162) 7-(2-(3-fluoro-4-((7-(3-(4-methylpiperidin-1-yl)propoxy)quinolin-4-yl)oxy)phenyl)acetyl)-5-(4-fluorophenyl)-3,5-dihydrofuro[3,2-c]pyridin-6(2H)-one;
163) N-(3-fluoro-4-((6-(methylcarbamoyl)-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
164) N-(3 -fluoro-4-((6-(methylcarbamoyl)-7-(3 - morpholinopropoxy)quinolin-4-yl)oxy)phenyl)-5-(4-fluorophenyl)-6-oxo-2,3,5,6-tetrahydrofuro[3,2-c]pyridine-7-carboxamide;
165) N-(3-fluoro-4-((6-methoxy-7-(methylcarbamoyl)quinolin-4-yl)oxy)phenyl)-5-(4-fluorophenyl)-6-oxo-2,3,5,6-tetrahydrofuro[3,2-c]pyridine-7-carboxamide;
166) N-(3-fluoro-4-((7-(methylcarbamoyl)-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
167) N-(3-fluoro-4-((6-fluoro-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
168) N-(3-fluoro-4-((6-fluoro-7-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
169) N-(3-fluoro-4-((7-(2-morpholinoethoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
170) N-(3-fluoro-4-((7-(3-morpholinopropoxy)-6-(trifluoromethyl)quinolin-4-yl)oxy)phenyl)-5-(4-fluorophenyl)-6-oxo-2,3,5,6-tetrahydrofuro[3,2-c]pyridine-7-carboxamide;
171) N-(4-((6-chloro-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
172) N-(4-((6-chloro-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
173) N-(4-((6-cyano-7-(2-morpholinoethoxy)quinolin-4-yl)oxy)-3-fluorophenyl)-5-(4-fluorophenyl)-6-oxo-2,3,5,6-tetrahydrofuro[3,2-c]pyridine-7-carboxamide;
174) N-(4-((6-cyano-7-(3-morpholinopropoxy)quinolin-4-yl)oxy)-3-fluorophenyl)-5-(4-fluorophenyl)-6-oxo-2,3,5,6-tetrahydrofuro[3,2-c]pyridine-7-carboxamide;
175) N-(3-fluoro-4-((7-methoxy-6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
176) N-(3-fluoro-4-((7-methoxy-6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
177) N-(3-fluoro-4-((6-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide;
178) N-(3-fluoro-4-((6-(3-morpholinopropoxy)quinolin-4-yl)oxy)phenyl)-5-(4-fluorophenyl)-6-oxo-2,3,5,6-tetrahydrofuro[3,2-c]pyridine-7-carboxamide;
179) N-(4-((6-amino-7-methoxyquinolin-4-yl)oxy)-3-fluorophenyl)-5-(4-fluorophenyl)-6-oxo-2,3,5,6-tetrahydrofuro[3,2-c]pyridine-7-carboxamide;
180) N-(3-fluoro-4-((7-methoxy-6-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
181) N-(4-((7-amino-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
182) N-(3-fluoro-4-((6-methoxy-7-(2-morpholinoacetamido)quinolin-4-yl)oxy)phenyl)-5-(4-fluorophenyl)-6-oxo-2,3,5,6-tetrahydrofuro[3,2-c]pyridine-7-carboxamide;
183) N-(3-fluoro-4-((6-methoxy-7-(3-morpholinopropanamido)quinolin-4-yl)oxy)phenyl)-5-(4-fluorophenyl)-6-oxo-2,3,5,6-tetrahydrofuro[3,2-c]pyridine-7-carboxamide;
184) N-(4-((7-acetamido-6-methoxyquinolin-4-yl)oxy)-3-fluorophenyl)-5-(4-fluorophenyl)-6-oxo-2,3,5,6-tetrahydrofuro[3,2-c]pyridine-7-carboxamide;
185) N-(3-fluoro-4-((7-methoxy-6-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide;
186) N-(3-fluoro-4-((7-methoxy-6-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide;
187) N-(3-fluoro-4-((6-methoxy-7-((2-morpholinoethyl)amino)quinolin-4-yl)oxy)phenyl)-5-(4-fluorophenyl)-6-oxo-2,3,5,6-tetrahydrofuro[3,2-c]pyridine-7-carboxamide; and
188) N-(3-fluoro-4-((6-methoxy-7-((3-morpholinopropyl)amino)quinolin-4-yl)oxy)phenyl)-5-(4-fluorophenyl)-6-oxo-2,3,5,6-tetrahydrofuro[3,2-c]pyridine-7-carboxamide.

Preferably, the compounds of Formula 2 may be
N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide; or
N-(4-((7-(3 -(3 -cyanoazetidin-1 -yl)propoxy)-6-methoxyquinolin-4-yl)oxy)-3 - fluorophenyl)-5-(4-fluorophenyl)-6-oxo-2,3,5,6-tetrahydrofuro[3,2-c]pyridine-7-carboxamide.

Hereinafter, a method for preparing the compound of Formula 2 will be described. The compound of Formula 2 can be prepared using a method as shown by the following schemes. However, the present invention is not limited to a case where the compound is prepared by this method. In particular, those skilled in the art will be able to fully understand that the compound of Formula 2 of the present invention can be prepared by various methods using techniques well known in the art.

The following schemes show, in respective preparation steps, methods for preparing the compound of Formula 2, and the compound of Formula 2 may be prepared by changing reagents and solvents used in the following preparation steps or by changing the order of reactions.

According to an embodiment, the compound of Formula 2 may be prepared according to the procedures in Schemes 1 and 2.

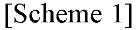

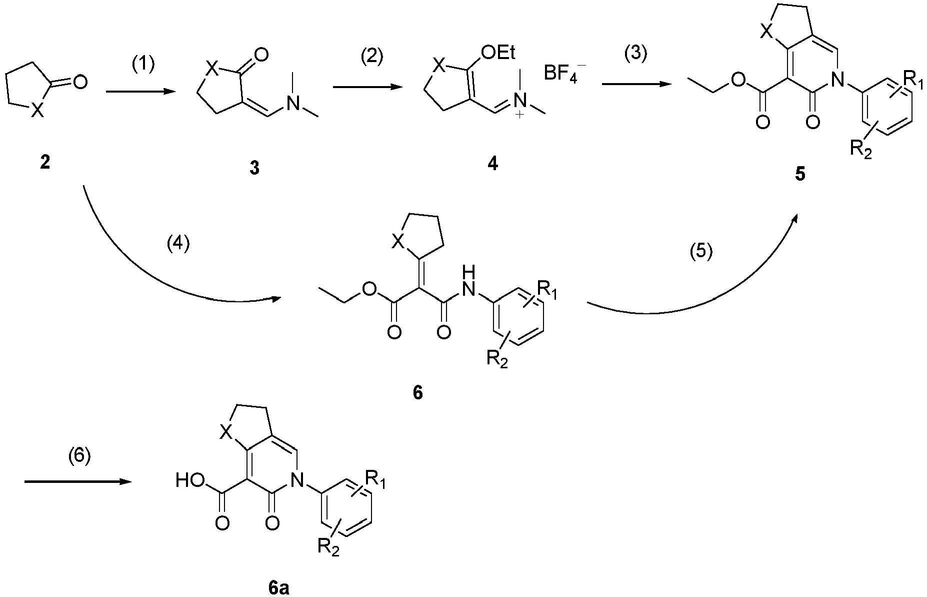

In Scheme 1, R₁, R₂, and X are as defined above in Formula 2.

According to Scheme 1, a carboxylic acid compound (6a) is prepared using, as a starting material, a lactone-based compound (2) that can be easily obtained commercially or is prepared by a known method.

Regarding Scheme 1, detailed description of each step is as follows.

In step 1, a compound (2), which can be easily obtained commercially, is subjected to formylation reaction using dimethyldimethoxyacetal, to prepare a compound (3). The reaction may generally be carried out at a high temperature; however, this is disadvantageous in that a long reaction time is required. Thus, the reaction is carried out using a microwave reactor.

In step 2, a compound (4) is prepared using the formylated lactone compound (3) in step 1 and triethyloxonium tetrafluoroborate which can be easily obtained commercially. This reaction is carried out under anhydrous conditions and is preferably carried out using a solvent, which does not adversely affect the reaction, such as N,N-dichloromethane or chloroform. For the reaction temperature, the reaction is generally carried out at room temperature. In step 3, the compound (4) prepared in step 2 is reacted with an ethyl-3-amino-3-oxopropionate compound, which has been prepared by a known method, in the presence of sodium ethoxide, to prepare a cyclized compound (5). This reaction is preferably carried out using an ethanol solvent that does not adversely affect the reaction. The reaction temperature is not particularly limited. The reaction may generally be carried out at a cold to warm temperature, and is preferably carried out at room temperature.

Alternatively, to prepare the cyclized compound (5), as in step 4, the lactone-based compound (2), which can be easily obtained commercially and is used as a starting material, may be reacted with an ethyl-3-amino-3-oxopropionate compound, which has been prepared by a known method, in the presence of titanium tetrachloride and pyridine, to prepare compound (6). This reaction is preferably carried out using dichloromethane that does not adversely affect the reaction. The reaction temperature is not particularly limited; and the reaction may generally be carried out at a cold to room temperature, and preferably starting from a cold temperature up to room temperature.

Thereafter, in step 5, the compound (6) prepared in step 4 is subjected to formylation and cyclization using dimethyldimethoxyacetal, to prepare the compound (5). The reaction may be generally carried out at a warm or high temperature, and is preferably carried out at a warm temperature.

In step 6, the cyclized compound (5) prepared in steps 3 and 5 is subjected to hydrolysis, to prepare the carboxylic acid compound (6a). In general, the hydrolysis is carried out using a basic aqueous solution such as an aqueous sodium hydroxide solution or an aqueous lithium hydroxide solution. This reaction is carried out using a solvent, which does not adversely affect the reaction, such as ethanol, methanol, or tetrahydrofuran, in the presence of an aqueous lithium hydroxide solution that can be used in the hydrolysis. The reaction temperature is not particularly limited. The reaction may generally be carried out at room temperature or a warm temperature, preferably at a warm temperature, to prepare the carboxylic acid compound (6a).

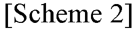

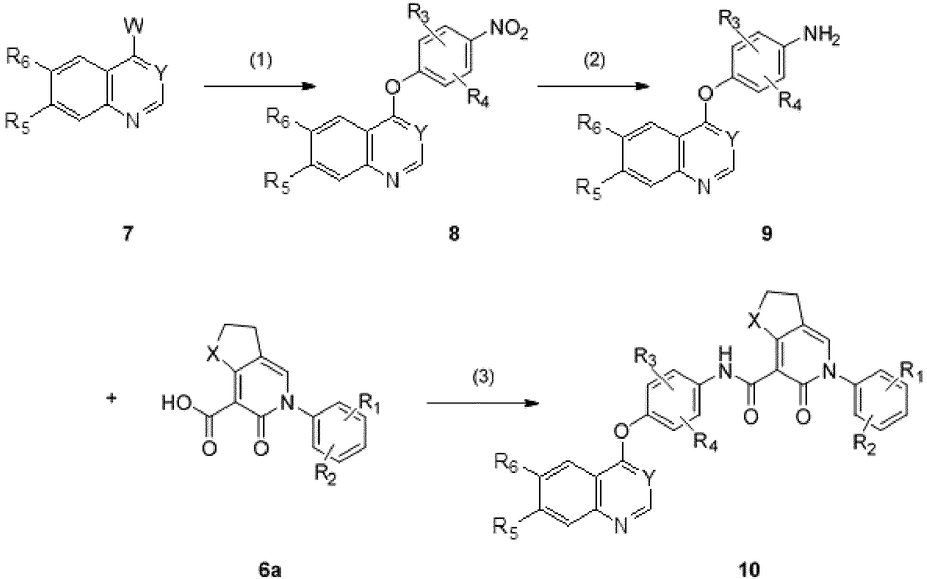

In Scheme 2, R₁ to R₆, X, and X are as defined in Formula 2, and W is a leaving group.

Scheme 2 specifically shows each step for preparing a desired compound (10) of the present invention.

In step 1, a monocyclic or bicyclic compound (7), which can be easily obtained commercially or prepared by a known method, is reacted with a nitrophenol compound, which can be easily obtained commercially, in the presence of a base such as potassium carbonate, to obtain a phenoxy compound (8). This reaction is generally etherification of a phenol compound and is carried out in the presence of a base that can be used for etherification. Examples of the base that can be used for this purpose include sodium hydrate (NaH), potassium carbonate, sodium carbonate, cesium carbonate, sodium or potassium alkoxide, and the like. In addition, the reaction is preferably carried out in the presence of a solvent that does not adversely affect the reaction. For example, the reaction is carried out using a solvent such as dichloromethane, chloroform, tetrahydrofuran, diethyl ether, toluene, N,N-dimethylformamide, acetonitrile, or diphenyl ether. The reaction temperature is not particularly limited. The reaction may generally be carried out at room temperature or a warm temperature, preferably at a warm temperature.

In step 2, the nitrophenol compound (8) prepared in step 1 is subjected to reduction in the presence of iron and ammonium chloride, to prepare an amine compound (9). This reaction is generally reduction of a nitro compound to an amine, and may be carried out using various reducing agents such as hydrogen, iron, tin(II) chloride, and zinc. In addition, for this reaction, a solvent, which does not adversely affect the reaction, is used, such as dichloromethane, ethyl acetate, methanol, ethanol, tetrahydrofuran, or N,N-dimethylformamide. As the case may be, the reaction is carried out using water as a co-solvent. The reaction temperature is not particularly limited. The reaction may generally be carried out at room temperature or a warm temperature, preferably at a warm temperature.

In step 3, a common amidation reaction is carried out, in which the amine compound (9) prepared in step 2 is reacted with the carboxylic acid compound (6a) prepared in Scheme 1 using a coupling reagent, to prepare the desired compound (10). In general, the reaction is carried out using a coupling reagent, which can be easily obtained commercially, such as 1-(3-dimethylaminopropyl)-3-ethylcarbodiimide (EDC), 1,3-dicyclohexyl carbodiimide (DCC), 1,1-carbonyl diimidazole (CDI), or 1-[bis(dimethylamino)methylene]-1H-1,2,3-triazolo[4,5-b]pyridinium 3-oxide hexafluorophosphate (HATU). This reaction may be carried out without using a base; however, the reaction is carried out using a solvent, which does not adversely affect the reaction, such as acetonitrile, dimethylformamide, or dichloromethane, in the presence of a common base, which can be used for an amidation reaction, such as 4-dimethylaminopyridine, pyridine, triethylamine, diethylisopropylamine, N-methylmorpholine, or dimethylphenylamine. The reaction temperature is not particularly limited. The reaction may generally be carried out at room temperature or a warm temperature, preferably at a warm temperature, to prepare the desired compound (10).

The desired compounds produced in the schemes can be separated and purified using conventional methods, for example, column chromatography, recrystallization, and the like.

As used herein, the term "halogen" refers to F, Cl, Br, or I unless otherwise stated.

The term "alkyl", unless otherwise specified, refers to a linear or branched saturated hydrocarbon moiety. For example, "C₁₋₁₀ alkyl" refers to an alkyl group having a skeleton of 1 to 10 carbon atoms. Specifically, the C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "haloalkyl" refers to an alkyl group substituted with one or more halogen atoms. Specifically, the haloalkyl may be an alkyl group substituted with two or more halogen atoms, the halogen atoms being the same or different.

The term "alkoxy", unless otherwise specified, refers to a group having the formula -O-alkyl, in which the alkyl is an alkyl group as defined above and is attached to a parent compound through an oxygen atom. The alkyl portion of the alkoxy group may have 1 to 20 carbon atoms (that is, C₁-C₂₀ alkoxy), 1 to 12 carbon atoms (that is, C₁-C₁₂ alkoxy), or 1 to 6 carbon atoms (that is, C₁-C₆ alkoxy). Examples of suitable alkoxy groups include methoxy (-O-CH₃ or -OMe), ethoxy (-OCH₂CH₃ or -OEt), t-butoxy (-OC(CH₃)₃ or -O-tBu), and the like.

The term "aryl" refers to an aromatic hydrocarbon radical derived by the removal of one hydrogen atom from a single carbon atom of a parent aromatic ring system. For example, the aryl group may have 6 to 20 carbon atoms, 6 to 14 carbon atoms, or 6 to 10 carbon atoms.

The term "cycloalkyl" refers to a saturated monocycle or polycycle that contains only carbon atoms in the ring. The cycloalkyl may have 3 to 7 carbon atoms as a monocycle, 7 to 12 carbon atoms as a bicycle, and up to about 20 carbon atoms as a polycycle.

The term "heteroaryl" refers to an aromatic heterocyclyl having one or more heteroatoms in the ring. Non-limiting examples of the heteroaryl include pyridinyl, pyrrolyl, oxazolyl, indolyl, isoindolyl, furinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl, and the like (each of which may have one or more substituents on the ring).

The term "heterocycle" refers to an aromatic or non-aromatic ring having one or more heteroatoms, which may be saturated or unsaturated and may be monocyclic or polycyclic. For example, "4- to 10-membered heterocycle" refers to a heterocycle having a skeleton of a total of 4 to 10 atoms including at least one heteroatom and carbon atoms. Specifically, examples of the 4- to 10-membered heterocycle may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, and the like.

The term "heterocycloalkyl" refers to a non-aromatic heterocyclyl having one or more heteroatoms in the ring. The heterocycloalkyl may have one or more carbon-carbon double bonds or carbon-heteroatom double bonds in the ring as long as the ring is not rendered aromatic by their presence. Non-limiting examples of the heterocycloalkyl include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl, and the like (each of which may have one or more substituents on the ring).

The term "heteroatom" refers to an atom other than carbon (C), and may specifically be a nitrogen (N), oxygen (O), or sulfur (S) atom. The above-mentioned heteroaryl and heterocycloalkyl contain one or more heteroatoms and may, for example, contain 1, 1 to 2,1 to 3, or 1 to 4 heteroatoms.

The term "substitution" refers to replacement of a hydrogen atom in a molecular structure with a substituent such that a chemically stable compound results from the substitution while not exceeding valence on the designated atom. For example, "group A is substituted with substituent B" or "group A has substituent B" means that a hydrogen atom bonded to an atom, such as carbon, which constitutes a skeleton of group A, is replaced with substituent B so that the group A and the substituent B form a covalent bond. Thus, it is substantially difficult or impossible for a group having no removable hydrogen atom to have a substituent. From this viewpoint, in a case where a range of combinations of various groups, which include groups that hardly have a substituent, with substituents are exemplified in the present specification, it should be interpreted that combinations of the groups, for which it is obvious that no substitution is possible, with the substituents are excluded from the range.

The phamaceutical composition of the present invention may comprise a pharmaceutically acceptable salt of the compound represented by Formula 1 or Formula 2 as an active ingredient.

The pharmaceutically acceptable salt should have low toxicity to humans and should not have any negative effect on the biological activity and physicochemical properties of its parent compound.

For example, the pharmaceutically acceptable salt may be an acid addition salt formed with a pharmaceutically acceptable free acid.

As the free acid, an inorganic acid or an organic acid may be used, wherein the inorganic acid may be hydrochloric acid, sulfuric acid, nitric acid, phosphoric acid, perchloric acid, bromic acid, or the like, and the organic acid may be acetic acid, methane sulfonic acid, ethane sulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, or the like.

The acid addition salt may be prepared by a conventional method, for example, by dissolving the compound of Formula 1 or the compound of Formula 2 in an excess amount of acid aqueous solution, and causing the salt thus formed to precipitate using a water-miscible organic solvent such as methanol, ethanol, acetone, or acetonitrile.

In addition, the pharmaceutically acceptable salt may be an alkali metal salt (such as sodium salt) or an alkaline earth metal salt (such as potassium salt).

The alkali metal salt or the alkaline earth metal salt may be obtained, for example, by dissolving the compound of Formula 1 or the compound of Formula 2 in an excess amount of alkali metal hydroxide or alkaline earth metal hydroxide solution, filtering the undissolved compound salt, and then evaporating and drying the filtrate.

In addition, the compounds of the present invention may have a chiral carbon center, and thus may exist in the form of R or S isomers, racemic compounds, individual enantiomers or a mixture thereof, or individual diastereomers or a mixture thereof. All such stereoisomers and mixtures thereof may be included in the scope of the present invention.

In addition, the compound of the present invention may include hydrates and solvates of the compound of Formula 1 or the compound of Formula 2. The hydrates and the solvates may be prepared using known methods, and are preferably non-toxic and water-soluble. In particular, the hydrates and the solvates may preferably be those obtained by binding, to the compound, 1 to 5 molecules of water and alcoholic solvent (in particular, ethanol or the like), respectively.

The phamaceutical composition of the present invention may comprise, as an active ingredient, the compound represented by Formula 1 or Formula 2 or a pharmaceutically acceptable salt thereof in an amount of 0.1% to 90% by weight, specifically 0.5% to 75% by weight, and more specifically 1% to 50% by weight, with respect to a total weight of the composition.

The phamaceutical composition of the present invention may comprise conventional and non-toxic pharmaceutically acceptable additives which are blended into a preparation according to a conventional method. For example, the pharmaceutical composition may further comprise a pharmaceutically acceptable carrier, diluent, or excipient.

Examples of the additives used in the composition of the present invention may include sweeteners, binders, solvents, dissolution aids, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The composition of the present invention may be made into various preparation forms for oral administration (for example, tablets, pills, powders, capsules, syrups, or emulsions) or parenteral administration (for example, intramuscular, intravenous, or subcutaneous injection).

Preferably, the composition of the present invention may be made into a preparation for oral administration, in which additives used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like.

Specifically, solid preparations for oral administration include tablets, pills, powders, granules, capsules, and the like, and such solid preparations can be formulated by mixing the composition with at least one excipient, such as starch, calcium carbonate, sucrose, lactose, and gelatin. In addition, in addition to simple excipients, a lubricant such as magnesium stearate and talc may be used.

In addition, as liquid preparations for oral administration, suspensions, emulsions, syrups, and the like may be exemplified, in which in addition to water and liquid paraffin which are commonly used simple diluents, various excipients, such as wetting agents, sweeteners, fragrances, and preservatives, may be included therein.

In addition, preparations for parenteral administration include sterilized aqueous solutions, non-aqueous solutions, suspensions, emulsions, lyophilized preparations, and suppositories. As the non-aqueous solution or the suspension, propylene glycol, polyethylene glycol, vegetable oil such as olive oil, injectable ester such as ethyl oleate, or the like may be used. As a base for the suppository, Witepsol, Macrogol, and Tween 61, cocoa butter, laurin fat, glycerogelatin, or the like may be used. On the other hand, the injection may comprise conventional additives such as solubilizing agents, isotonic agents, suspending agents, emulsifying agents, stabilizing agents, and preservatives.

The compound or pharmaceutical composition of the present invention may be administered to a patient in a therapeutically effective amount or a pharmaceutically effective amount.

Here, the "therapeutically effective amount" or "pharmaceutically effective amount" refers to an amount of a compound or composition effective to prevent or treat a target disease, the amount being sufficient to treat the disease at a reasonable benefit/risk ratio, which is applicable to medical treatment, without causing adverse effects. A level of the effective amount may be determined depending on factors, including the patient's health status, disease type, disease severity, drug activity, drug sensitivity, method of administration, time of administration, route of administration and rate of excretion, duration of treatment, and drugs used simultaneously or in combination, and other factors well known in the medical field.

The compound or pharmaceutical composition of the present invention may be administered as an individual therapeutic agent or administered in combination with other therapeutic agents, may be administered sequentially or simultaneously with a conventional therapeutic agent, and may be administered once or multiple times. It is important to administer an amount such that a maximum effect can be obtained with a minimum amount without adverse effects by taking all of the above-described factors into consideration, and such an amount can be readily determined by those skilled in the art.

Specifically, an effective amount of the compound comprised in the pharmaceutical composition may vary depending on the patient's age, sex, and body weight. Generally, the compound may be administered in an amount of 0.1 mg to 1,000 mg, or 5 mg to 200 mg, per kg body weight, on a daily or every-other-day basis, or once or three times a day. However, the effective amount may increase or decrease depending on route of administration, disease severity, the patient's sex, body weight, and age, and the like. Thus, the scope of the present invention is not limited thereto.

Preferably, the compound or pharmaceutical composition of the present invention may be administered for tumor therapy, in combination with chemotherapy, radiation therapy, immunotherapy, hormone therapy, bone marrow transplantation, stem cell replacement therapy, other biological therapies, surgical intervention, or a combination thereof. For example, the compound or composition of the present invention may be used as adjuvant therapy in combination with other long-term treatment strategies, or may be used to maintain the patient's condition after tumor regression is induced or chemoprophylactic therapy is applied in severely ill patients.

Phamaceutical composition of the present invention may additionally comprise one or more active ingredients, and the additional active ingredient may be, but is not limited to, an anti-proliferative compound such as an aromatase inhibitor, anti-estrogen, a topoisomerase I inhibitor, a topoisomerase II inhibitor, a microtubule active compound, an alkylated compound, a histone deacetylase inhibitor, a compound that induces cell differentiation processes, a cyclooxygenase inhibitor, an MMP inhibitors, an mTOR inhibitor, an anti-neoplastic anti-metabolite, a platinum compound, a compound that targets or decreases protein activity or lipid kinase activity, an anti-angiogenic compound, a compound that targets, decreases, or inhibits protein activity or lipid phosphatase activity, a gonadorelin agonist, anti-androgen, a methionine aminopeptidase inhibitor, bisphosphonate, a biological response modifier, an anti-proliferative antibody, a heparanase inhibitor, an inhibitor of Ras oncogenic isoforms, a telomerase inhibitor, a proteasome inhibitor, a compound used for treatment of hematologic malignancies, a compound that targets, decreases, or inhibits Flt-3 activity, an HSP90 inhibitor, a kinesin spindle protein inhibitor, a MEK inhibitor, leucovorin, an EDG binder, an anti-leukemic compound, a ribonucleotide reductase inhibitor, an S-adenosylmethionine decarboxylase inhibitor, a hemostatic steroid, a corticosteroid, another chemotherapy compound, or a photosensitizing compound.

Preferably, the additional active ingredient may be a known anticancer agent. Non-limiting examples of the anticancer agent include DNA alkylating agents, such as mechlorethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anti-cancer antibiotics, such as dactinomycin (actinomycin D), doxorubicin (adriamycin), daunorubicin, idarubicin, mitoxantrone, plicamycin, mitomycin C, and bleomycin; and plant alkaloids, such as vincristine, vinblastine, paclitaxel, docetaxel, etoposide, teniposide, topotecan, and irinotecan; and the like.

### Mode for the Invention

Hereinafter, the present invention will be described in more detail by way of the following examples. However, the following examples are for illustrative purposes only, and the scope of the present invention is not limited thereto.

### Preparation Example 1: 5-(4-Fluorophenyl)-6-oxo-2,3,5,6-tetrahydrofuro[3,2-c]pyridine-7-carboxylic acid

### Step 1: Synthesis of 3-((dimethylamino)methylene)-dihydrofuran-2-(3H)-one

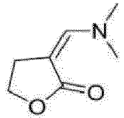

Gamma-butyrolactone (2.69 mL, 35.0 mmol) and dimethyldimethoxyacetal (11.62 mL, 87.5 mmol) were stirred, and then reacted in a microwave reactor at a temperature of 230°C for 70 minutes or longer. The reaction mixture was concentrated to remove excess dimethyldimethoxyacetal. Solidification was performed with diethyl ether, and the precipitated solid was filtered while washing with diethyl ether. The filtered solid was concentrated under reduced pressure to obtain the title compound (2.9 g, yield: 59%, brownish solid).

¹H NMR (500 MHz, CDCl₃) δ 7.13 (s, 1H), 4.24 (t, J = 7.5 Hz, 2H), 3.11 (t, J = 7.5 Hz, 2H), 3.03 (s, 6H); ¹H NMR (500 MHz, DMSO-d₆) δ 7.00 (t, J = 1.5 Hz, 1H), 4.11 (t, J = 7.5 Hz, 2H), 3.06 (t, J = 7.5 Hz, 2H), 3.00 (s, 3H)

### Step 2: Synthesis of 3-((dimethylamino)methylene)-2-(3H)-dihydrofuranilidene ethyl oxonium tetrafluoroborate

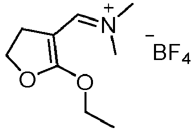

The compound (1.085 g, 7.68 mmol) obtained in step 1 was dissolved in 8 ml of chloroform. Then, triethyloxonium tetrafluoroborate (1.729 g, 7.68 mmol) was added thereto and stirred under nitrogen condition at room temperature for 1 day or longer. The reaction mixture was concentrated under reduced pressure. Nuclear magnetic resonance was used to identify that the starting material and the resulting material were produced in a ratio of about 15:85, and the next reaction was performed without purification.

¹H NMR (500 MHz, DMSO-d₆) δ 7.93 (s, 1H), 4.86-4.82 (m, 2H), 4.51 (q, J = 7.0 Hz, 2H), 3.33 (s, 3H), 3.30 (t, J = 8.5 Hz, 2H), 3.26 (s, 3H), 1.36 (t, J = 7.0 Hz, 3H)

### Step 3: Synthesis of ethyl 5-(4-fluorophenyl)-6-oxo-2,3,5,6-tetrahydrofuro[3,2-c]pyridine-7-carboxylate

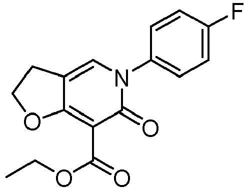

The crude mixture (1.96 g) obtained in step 2 was dissolved in 10 ml of ethanol. Subsequently, sodium ethoxide (20 wt% solution in ethanol, 2.56 ml, 6.53 mmol) was added thereto in a water bath at 0°C, and then slowly stirred for 30 minutes to room temperature. To the reaction mixture was added ethyl 3-((4-fluorophenyl)amino)-3-oxopropionate (1.47 g, 6.53 mmol), and stirrring was performed at room temperature for 20 hours or longer. The reaction mixture was concentrated under reduced pressure and extracted with water and dichloromethane. The separated organic layer was dried over anhydrous magnesium sulfate, filtered, and concentrated under reduced pressure. The resulting residue was purified by column chromatography to obtain the title compound (900 mg, yield: 39% (based on overall yield of steps 2 and 3)/46% (based on yield of step 3), yellow solid).

¹H NMR (500 MHz, DMSO-d₆) δ 7.70 (t, J = 1.5 Hz, 1H), 7.42-7.40 (m, 2H), 7.34-7.31 (m, 2H), 4.74 (t, J = 8.0 Hz, 2H), 4.17 (q, J = 7.0 Hz, 2H), 3.05 (td, J = 8.0 Hz, 2H), 1.21 (t, J = 7.0 Hz, 3H)

### Step 4: Synthesis of 5-(4-fluorophenyl)-6-oxo-2,3,5,6-tetrahydrofuro[3,2-c]pyridine-7-carboxylic acid

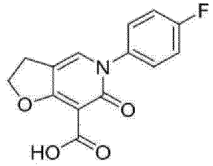

The compound (0.9 g, 2.97 mmol) obtained in step 3 was dissolved in 10 ml of ethanol and 5 ml of distilled water, and then lithium hydroxide monohydrate (249 mg, 5.94 mmol) was added thereto. The mixture was heated to a temperature of 50°C and stirred for 4 hours or longer. The reaction mixture was concentrated under reduced pressure and extracted with water and dichloromethane. 1 N hydrochloric acid solution was added to the separated water layer, and then the organic layer was extracted with water and dichloromethane. The separated organic layer was dried over anhydrous magnesium sulfate, filtered, and concentrated under reduced pressure. The concentrated residue was treated with a small amount of dichloromethane and diethyl ether to precipitate a solid, and filtration was performed. Then, the filtered solid was dried to obtain the title compound (680 mg, yield: 84%, off-white solid).

¹H NMR (500 MHz, DMSO-d₆) δ 14.5 (bs, OH), 7.97 (s, 1H), 7.54-7.51 (m, 2H), 7.41-7.37 (m, 2H), 4.90 (t, J = 8.5 Hz, 2H), 3.11 (td, J = 8.5, 1.0 Hz, 2H)

### Example 1: 4-ethoxy-N-[3-fluoro-4-({2-[5-(morpholinomethyl)pyridine-2-yl]thieno[3,2-b]pyridine-7-yl}oxy)phenyl]-1-(4-fluorophenyl)-2-oxo-1,2-dihydropyridine-3-carboxamide hydrochloride

The title compound was synthesized by a method described in Example 31 of Korean Patent Laid-open Publication No. 2019-0106802.

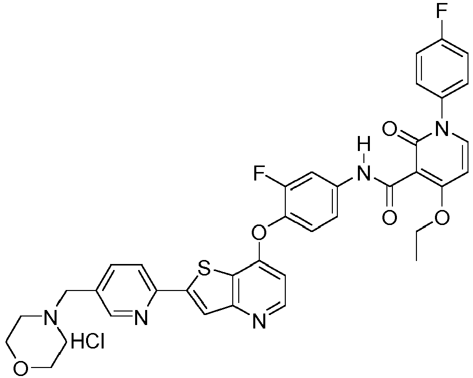

¹H NMR (500MHz, DMSO-d₆) δ 10.66 (s, 1H), 8.81 (s, 1H), 8.60 (d, J = 5.0 Hz, 1H), 8.48 (s, 1H), 8.43 (d, J = 5.0 Hz, 1H), 8.23 (d, J = 5.0 Hz, 1H), 7.96 (d, J = 15.0 Hz, 1H), 7.87 (d, J = 5.0 Hz, 1H), 7.52-7.45 (m, 4H), 7.39-7.36 (m, 2H), 6.83 (d, J = 5.0 Hz, 1H), 6.53 (d, J = 10.0 Hz, 1H), 4.44 (s, 2H), 4.26 (qt, J = 5.0 H, 2H), 3.96-3.94 (m, 2H), 3.77 (t, J = 10.0 Hz, 2H), 3.32-3.30 (m, 2H), 3.14 (qt, J = 10.0 2H), 1.31 (t, J = 5.0 Hz, 3H)

### Example 2: 4-ethoxy-N-(3-fluoro-4-{[2-(5-{[(2-methoxyethyl)amino]methyl}pyridine-2-yl)thieno[3,2-b]pyridine-7-yl]oxylphenyl)-2-oxo-1-phenyl-1,2-dihydropyridine-3-carboxamide hydrochloride

The title compound was synthesized by a method described in Example 1 of Korean Patent Laid-open Publication No. 2019-0106802.

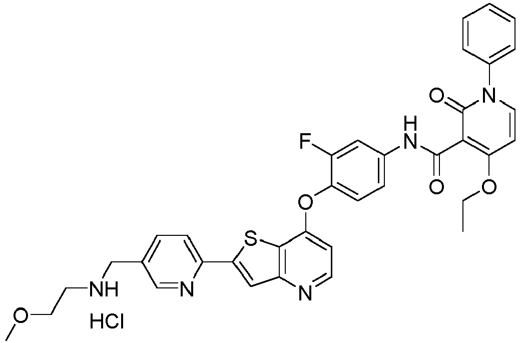

¹H NMR (500 MHz, DMSO-d₆) δ 10.70 (brs, 1H), 9.50 (brs, 2H), 8.80 (s, 1H), 8.69 (d, J = 5.0 Hz, 1H), 8.47 (s, 1H), 8.44 (d, J = 5.0 Hz, 1H), 8.22 (dd, J = 10.0 and 5.0 Hz, 1H), 7.99 (d, J = 10.0 Hz, 1H), 7.88 (d, J = 5.0 Hz, 1H), 7.57-7.40 (m, 7H), 6.97 (d, J = 5.0 Hz, 1H), 6.53 (d, J = 5.0 Hz, 1H), 4.29-4.25 (m, 4H), 3.65 (t, J = 5.0 Hz, 2H), 3.31 (s, 3H), 3.16-3.12 (m, 2H), 1.31 (t, J = 5.0 Hz, 3H)

### Example 3: 4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide hydrochloride

The title compound was synthesized by a method described in Example 34 of Korean Patent Laid-open Publication No. 2019-0106802.

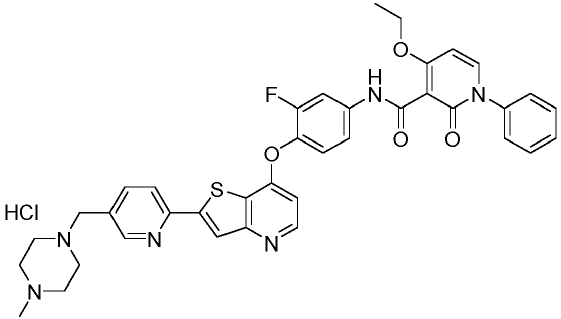

¹H NMR (500MHz, DMSO-d₆) δ 10.68 (s, 1H), 8.82 (brs, 1H), 8.61 (d, J = 5.0 Hz, 1H), 8.47 (s, 1H), 8.41 (d, J = 5.0 Hz, 1H), 8.23 (s, 1H), 7.97 (d, J = 15.0 Hz, 1H), 7.87 (d, J = 5.0 Hz, 1H), 7.56-7.46 (m, 5H), 7.42-7.40 (m, 2H), 6.86 (d, J = 5.0 Hz, 1H), 6.52 (d, J = 5.0 Hz, 1H), 4.26 (qt, J = 5.0 H, 2H), 3.93 (brs, 8H), 3.58 (brs, 2H), 2.81 (s, 3H), 1.31 (t, J = 5.0 Hz, 3H)

### Example 4: N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

### Step 1: Synthesis of 4-(2-((4-chloro-6-methoxyquinolin-7-yl)oxy)ethyl)morpholine

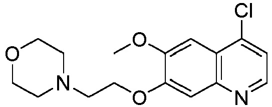

4-Chloro-6-methoxyquinoline-7-ol (500 mg, 2.39 mmol) was dissolved in dimethylformamide. Then, cesium carbonate (2.33 g, 7.16 mmol), sodium iodide (536 mg, 3.58 mmol), and 4-((2-chloroethyl)morpholine) hydrochloride (666 mg, 3.58 mmol) were sequentially added thereto, and stirred overnight at a temperature of 60°C. Subequently, the resulting mixture was extracted with ethyl acetate and water, dried over anhydrous sodium sulfate, filtered, and concentrated under reduced pressure. The resulting residue was purified by column chromatography to obtain the title compound (310 mg, yield: 40%, ivory solid).

¹H NMR (500 MHz, CDCl₃) δ 8.57(d, J = 5.2 Hz, 1H), 7.40(d, J = 6.0 Hz, 2H), 7.35(d, J = 5.2 Hz, 1H), 4.33 (t, J = 6.4Hz, 2H), 4.02(s, 3H), 3.74 (t, J = 4.8Hz, 4H), 2.95 (t, J = 6.0Hz, 2H), 3.74 (t, J = 5.2Hz, 4H) δ 8.58 (d, J = 5.0 Hz, 1H), 7.42 (s, 1H), 7.40 (s, 1H), 7.36 (d, J = 5.0 Hz, 1H), 4.28 (t, J = 5.0 Hz, 2H), 4.03 (s, 3H), 3.85 (m, 4H), 2.65 (m, 4H), 2.32 (m, 2H), 2.15 (m, 2H)

### Step 2: Synthesis of 4-(2-((4-(2-fluoro-4-nitrophenoxy)-6-methoxyquinolin-7-yl)oxy)ethyl)morpholine

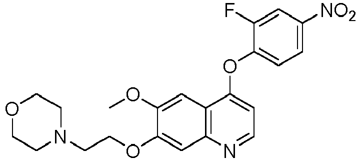

The compound (310 mg, 0.96 mmol) prepared in step 1 was dissolved in diphenyl ether. Then, anhydrous potassium carbonate (199 mg, 1.44 mmol) and 2-fluoro-4-nitrophenol (302 mg, 1.92 mmol) were sequentially added thereto, and stirred overnight at a temperature of 220°C. Subsequently, the reaction mixture was cooled to room temperature, extracted with ethyl acetate and water, dried over anhydrous sodium sulfate, filtered, and concentrated under reduced pressure. The resulting residue was purified by column chromatography to obtain the title compound (362 mg, yield: 85%, yellow solid).

¹H NMR (500 MHz, DMSO-d₆) δ 8.57(d, J = 5.5 Hz, 1H), 8.47-8.45(dd, J = 3.0 Hz, J = 10.5 Hz, 1H), 8.21(m, 1H), 7.63 (t, J = 8.5Hz, 1H), 7.48(s, 1H), 7.45(s, 1H), 6.78(d, J = 5.0 Hz, 1H), 4.30 (t, J = 5.5Hz, 2H), 3.92(s, 3H), 3.60 (t, J = 4.5Hz, 4H), 2.80 (t, J = 5.5Hz, 2H), 2.52-2.49 (m, 4H, partially overlapped with DMSO)

### Step 3: Synthesis of 3-fluoro-4-((6-methoxy-7-(2-morpholinoethoxy)quinolin-4-yl)oxy)aniline

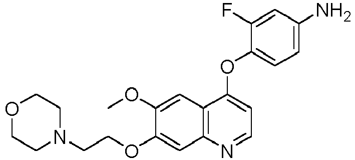

The compound (360 mg, 0.81 mmol) prepared in step 2 was dissolved in ethanol and water. Then, iron (136 mg, 2.43 mmol) and ammonium chloride (433 mg, 8.10 mmol) were sequentially added thereto at room temperature, heated to a temperature of 80°C, and stirred for 4 hours. After completion of the reaction, the reaction mixture was filtered using a celite pad and concentrated under reduced pressure. The resulting residue was extracted with dichloromethane and water. The separated organic layer was dried over anhydrous sodium sulfate, concentrated under reduced pressure, and then filtered with hexane to obtain the title compound (280 mg, yield: 83%, yellow solid).

¹H NMR (500 MHz, DMSO-d₆) δ 8.45 (d, J = 5.0 Hz, 1H), 7.50 (s, 1H), 7.41 (s, 1H), 7.07 (t, J = 10.0 Hz, 1H), 6.54 (d, J = 10.0 Hz, 1H), 6.47 (d, J = 10.0 Hz, 1H), 6.38 (d, J = 5.0 Hz, 1H), 5.50 (brs, 2H), 4.27 (t, J = 10.0 Hz, 2H), 3.94 (s, 3H), 3.60 (t, J = 5.0 Hz, 4H), 2.79 (brs, 2H) 2.52 (brs, 4H, partially overlapped with DMSO)

### Step 4: Synthesis of N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide

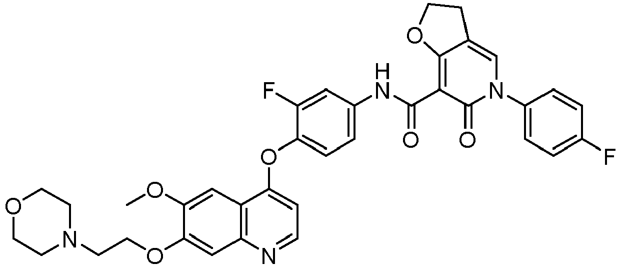

The compound (149 mg, 0.54 mmol) prepared in Preparation Example 1 was dissolved in dimethylformamide. Then, 1-[bis(dimethylamino)methylene)-1H-1,2,3-triazolo[4,5-b]pyridinium 3-oxide hexafluorophosphate (HATU, 205 mg, 0.54 mmol), diisopropylethylamine (293 µl, 1.80 mmol), and the compound (150 mg, 0.36 mmol) prepared in step 3 were sequentially added thereto, and stirred overnight at room temperature. Subsequently, the reaction mixture was extracted with ethyl acetate and a saturated aqueous sodium bicarbonate solution, dried over anhydrous sodium sulfate, filtered, and concentrated under reduced pressure. The resulting residue was purified by column chromatography to obtain the title compound (170 mg, yield: 70%, white solid).

¹H NMR (500 MHz, DMSO-d₆) δ 11.89 (s, 1H), 8.48 (d, J = 5.0 Hz, 1H), 8.00 (d, J = 10.0 Hz, 1H), 7.88 (s, 1H), 7.54-7.49 (m, 3H), 7.45-7.37 (m, 5H), 6.48 (d, J = 5.0 Hz, 1H), 4.85 (t, J = 10.0 Hz, 2H), 4.32 (brs, 2H), 3.95 (s, 3H), 3.63 (brs, 4H), 3.11 (t, J = 10.0 Hz, 2H), 2.52 (m, 2H, partially overlapped with DMSO), 2.50 (m, 4H, overlapped with DMSO)

### Example 5: N-(4-((7-(3-(3-cyanoazetidine-1-yl)propoxy)-6-methoxyquinoline-4-yl)oxy)-3-fluorophenyl)-5-(4-fluorophenyl)-6-oxo-2,3,5,6-tetrahydrofuro[3,2-c]pyridine-7-carboxamide

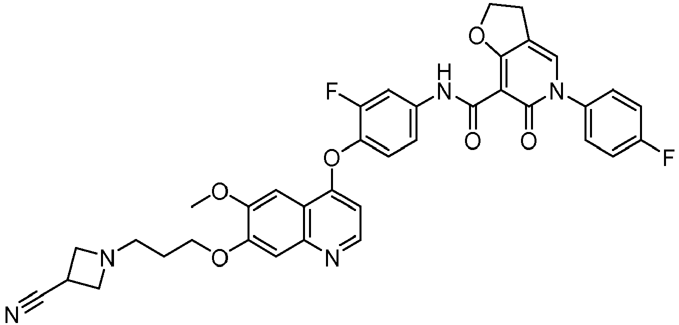

The title compound (yield: 57%, off-white solid) was obtained using the same synthetic route as in Example 4, except that azetidine-3-carbonitrile was used as a starting material in step 1.

¹H NMR (400 MHz, CDCl₃) δ 12.04 (S, 1H), 8.48 (d, J = 5.2 Hz, 1H), 8.03 (dd, J = 12.4, 2.4 Hz, 1H), 7.57 (s, 1H), 7.40-7.37 (m, 5H), 7.26-7.22 (m, 2H), 7.17 (t, J = 8.8 Hz, 1H), 6.44 (d, J = 5.6 Hz, 1H), 5.01 (t, J = 8.4 Hz, 2H), 4.26 (t, J = 6.4 Hz, 2H), 4.03 (s, 3H), 3.64 (t, J = 11.6 Hz, 4H), 3.20 (t, J = 8.0 Hz, 2H), 2.81 (t, J = 6.8 Hz, 2H), 2.05 (q, J = 6.8 Hz, 3H)

### Positive control 1

N-{4-[(2-amino-3-chloro-4-pyridinyl)oxy]-3-fluorophenyl}-4-ethoxy-1-(4-fluorophenyl)-2-oxo-1,2-dihydro-3-pyridinecarboxamide, corresponding to Compound 1 disclosed in U.S. Patent No. 8,536,200 B2 which is BMS-777607, a well known RON inhibitor, was used as the compound of positive control 1.

### Positive control 2

3-[(R)-1-(2,6-dichloro-3-fluorophenyl)-ethoxy]-5-[1-(piperidine-4-yl-1H-pyrazole-4-yl]-pyridine-2-amine, corresponding to Compound 1 disclosed in PCT International Publication WO 2013/017989 A1 which is crizotinib, a well known anticancer drug, was used as the compound of positive control 2.

### Positive control 3

N-(4-{[6,7-bis(methyloxy)quinoline-4-yl]oxy}phenyl)-N'-(4-fluorophenyl)cyclopropane-1,1-dicarboxamide, corresponding to Compound 1 disclosed in PCT International Publication WO 2014/165786 A1 which is cabozantinib, a well known anticancer drug, was used as the compound of positive control 3.

### Experimental Example 1. Characterization of cell lines that exhibit secondary resistance to cetuximab

In order to analyze whether RON and KRAS mutations have occurred in a cell line (parent) which is sensitive to cetuximab, and a cell line (Cet-R2) exhibiting secondary resistance to cetuximab, obtained from colorectal cancer cell line LIM1215, an experiment was conducted as follows. The LIM1215 cell line (Cet-R2) which exhibits secondary resistance to cetuximab was obtained through the process illustrated in the schematic diagram of FIG. 1.

A cell pellet of each of the cell line (parent) sensitive to cetuximab, and the cell line (Cet-R2) exhibiting secondary resistance to cetuximab, obtained from the colorectal cancer cell line LIM1215, was obtained. To each sample was added 1 mL of Trizol^{™} from Thermo Fisher Scientific Inc. Pipetting was performed to break up the cells. Then, 200 µL of chloroform was added thereto and gently mixed for 15 seconds. Subsequently, reaction was allowed to proceed at room temperature for 3 minutes. The sample obtained after completion of the reaction was centrifuged at 12,000 rpm for 15 minutes at 4 °C using a centrifuge, to obtain a clear supernatant. The supernatant was placed in a new 1.5 mL-tube, and 0.5 mL of isopropanol was added thereto and gently mixed for 15 seconds. Then, reaction was allowed to proceed at room temperature for 10 minutes.

The sample obtained after completion of the reaction was centrifuged at 12,000 rpm for 1 minute at 4 °C using a centrifuge, and it was identified that a white RNA pellet has been generated at the bottom of the tube. Then, the supernatant was removed and 1 mL of 75% ethanol was added thereto. Mixing was performed for 10 seconds using a vortex mixer. Then, the mixture was centrifuged at 12,000 rpm for 10 minutes at 4 °C, and it was identified that an RNA pellet has been generated. Then, the supernatant was removed, and the tube was turned over and dried at room temperature until the liquid disappeared.

The dried RNA pellet was dissolved in 50 µL to 100 µL of RNase-free water, and then a concentration of the extracted total RNA was measured using Nano-drop^{™} from Malcom, Co., Ltd., and 1 µL of total RNA and 1 µL of oligo dT were mixed in a new tube. Using a heat block or constant-temperature water bath, reaction was allowed to proceed at 70 °C for 5 minutes. Then, the reaction mixture was immersed in ice for cooling. Then, the mixture of total RNA and oligo dT was added to a cDNA synthesis kit tube (AccuPower^{™} CycleScript RT PreMix, BIONEER CORPORATION), and the final volume was adjusted to 20 µL with RNase-free water. Then, cDNA was synthesized with a polymerase chain reaction (PCR) instrument by setting the parameter to 60 minutes at 42 °C and 5 minutes at 94 °C.

1 µg of the synthesized cDNA was added to RT-PCR premix kit tube (AccuPower^{™} Gold Multiplex PCR PreMix, BIONEER CORPORATION). Each 1 µL of forward primer (10 pmol) and reverse primer (10 pmol) for RON exons 5-6 or RON exon 11 was added thereto, and the volume was adjusted to 20 µL with RNase-free water. The nucleotide sequences for the primers are as shown in Table 1 below.

**[Table 1]**

| **Primer** | **Nucleotide sequence** | **SEQ ID NO** |
|---|---|---|
| RON d5_6 sense primer (Sense primer) | 5'- GAGCTGGTCAGGTCACTAAAC -3' | SEQ ID NO: 3 |
| RON d5 6 antisense primer | 5'- CAGACACTCAGTCCCATTGAC -3' | SEQ ID NO: 4 |
| RON d11 sense primer (Sense primer) | 5'- ATCTGTGGCCAGCATCTAAC-3' | SEQ ID NO: 5 |
| RON d11 antisense primer | 5'- AAAGGCAGCAGGATACCAAG- 3' | SEQ ID NO: 6 |

Then, RT-PCR was performed with a PCR instrument. In the case of the primers for RON exons 5-6, the PCR instrument was set as follows: An initial denaturation step of 5 minutes at 94 °C, an amplification step of 35 cycles of 30 seconds at 94 °C, 30 seconds at 59 °C, and 45 seconds at 72 °C, followed by a final extension step of 10 minutes at 72 °C. In the case of the primer for RON exon 11, the PCR instrument was set as follows: An initial denaturation step of 5 minutes at 94 °C, an amplification step of 35 cycles of 30 seconds at 94 °C, 30 seconds at 58 °C, and 45 seconds at 72 °C, followed by a final extension step of 10 minutes at 72 °C.

1% to 1.5% agarose was dissolved in 1x TBE buffer (diluted with Milli-Q water), and then completely dissolved by heating in a microwave oven. Subsequently, ethidium bromide was added to become a final concentration of 0.2 µg/mL, and then the mixture was placed in an agarose gel plate mold and solidified. The solidified agarose gel was poured into an electrophoresis instrument, and then the electrophoresis instrument was filled with 1x TBE buffer. Each well was loaded with 5 µL of each of the RT-PCR products and was loaded with 3 µL of DNA size marker (BIONEER 100bp plus DNA ladder, BIONEER CORPORATION). Electrophoresis was performed for 35 minutes at 150 V

The gel after the electrophoresis was placed on a UV transilluminator and photographed using Gel-Doc. The RT-PCR band for each sample on the agarose gel was cut, and then placed in a new 1.5 mL-tube. 300 µL of QG buffer (Qiagen) per 100 µg of gel weight was added thereto and dissolution was performed in a heat block or constant-temperature water bath at 56 °C. Then, 100 µL of isopropanol per 300 µL of QG buffer (Qiagen) was mixed and vortexed for 3 seconds. The mixture was placed in a DNA extraction column, and centrifuged at 13,000 rpm for 1 minute at room temperature.

In the centrifugation process, the liquid exiting the column was removed and 650 µL of washing buffer (Qiagen) was added. Centrifugation was performed at 13,000 rpm for 1 minute at room temperature. The centrifuged column was placed on a new 1.5 mL-tube, 30 µL of elusion buffer (Qiagen) was added to the column, and centrifugation was performed at 13,000 rpm for 1 minute at room temperature. The gel-eluted PCR product thus obtained was analyzed by performing Sanger sequencing.

As a result, as shown in Table 2 below, cell lines that exhibit secondary resistance to cetuximab were identified as having RON and KRAS mutations (FIGS. 2A and 2B).

**[Table 2]**

| **LIM1215** | **KRAS** | **RON** |
|---|---|---|
| **Parent** | WT | WT |
| **Cet-R2** | G13D | △155 |

### Experimental Example 2. Analysis of efficacy on cell line exhibiting secondary resistance to cetuximab

The parent cell line LIM1215 and the cetuximab-resistant cell line R2 were counted, and each of them was seeded at 3×10⁵ cells in a 60 mm dish. After 24 hours, treatment with 1 µM of each of the compounds of Example 1, Example 2, Example 3, Example 4, Example 5, positive control 1 (BMS-777607), positive control 2 (crizotinib), and positive control 3 (cabozantinib) was performed. After 48 hours had elapsed since the treatment, the cells were collected, resuspended in 1× phosphate buffered saline (PBS), and stained with a trypan blue solution. The stained cells were counted using a hemocytometer. Cell counting was performed in duplicate, and the mean and the standard deviation were calculated.

As a result, it was confirmed that each of the cell lines was treated with the compound of Example 1, and thus a high cell death rate was observed in the cell line R2 exhibiting secondary resistance to cetuximab. On the other hand, it was confirmed that each of the cell lines was treated with the compound of positive control 1 or positive control 3, cell death was not induced, each of the cell lines was treated with the compound of positive control 2, an increased cell death rate was observed only in the parent cell line, and cell death was not induced in the cell line exhibiting secondary resistance to cetuximab (FIG. 3).

In addition, each cell line was treated with each of the compounds of Example 1, Example 2, Example 3, Example 4, and Example 5, cell death was effectively induced in the cell line R2 exhibiting secondary resistance to cetuximab. In particular, it was confirmed that the compounds of Examples 1 to 5 exhibited remarkably excellent cell death rates in the cell line R2 exhibiting secondary resistance to cetuximab (FIG. 4).

### Experimental Example 3. Analysis of action mechanism of compound of Example 1 on cell line exhibiting secondary resistance to cetuximab

The parent cell line LIM1215 and the cetuximab-resistant cell line R2 were counted. 2×10⁶ cells of each cell line were washed with 1x PBS once and then lysed with 1x RIPA lysis buffer. The cell lysate was reacted on ice for 20 minutes, and then centrifuged at 13,000 rpm for 20 minutes at 4 °C to obtain a supernatant. After proteins in the obtained supernatant were quantified using Bradford method, 20 µg of the protein was subjected to sodium-polyacrylamide gel electrophoresis (SDS-PAGE).

Transfer was performed using polyvinylidene fluoride (PVDF) membrane. Then, each of phospho-RON antibody (MyBiosource, Inc., cat# MBS462024; 1:500), RON antibody (Santa Cruz Biotechnology, cat# sc-374626; 1:1,000), phospho-EGFR antibody (Cell Signaling Technology, Inc., cat# 2234; 1: 1,000), EGFR antibody (Cell Signaling Technology, Inc., cat# 2232; 1: 1,000), phospho-Akt antibody (Cell Signaling Technology, Inc., cat# 4060; 1: 1,000), Akt antibody (Cell Signaling Technology, Inc., cat# 9272; 1: 1,000), phospho-ERK antibody (Cell Signaling Technology, Inc., cat# 4370; 1:1,000), ERK antibody (Cell Signaling Technology, Inc., cat# 4695; 1: 1,000), phospho-β-catenin antibody (Cell Signaling Technology, Inc., cat# 9561; 1: 1,000), β-catenin antibody (Cell Signaling Technology, Inc., cat# 8480; 1: 1,000), c-myc antibody (Santa Cruz Biotechnology, cat# sc-40; 1:500), and β-actin antibody (Santa Cruz Biotechnology, cat# sc-47778; 1:1,000) was diluted in 5% skim milk tris buffered saline with Tween 20 (TBS-T) solution and reacted at 4 °C overnight.

After completion of the reaction, washing with the TBS-T solution was performed three times for 10 minutes each. Subsequently, anti-mouse-HRP or anti-rabbit-HRP (Cell Signaling Technology, Inc., cat# 7074 or 7076; 1:2,000) was diluted in the 5% skim milk TBS-T solution, and then reacted at room temperature for 2 hours. Then, washing with the TBS-T solution was performed three times for 10 minutes each, and then development was performed using an ECL solution (GE Healthcare, cat# RPN2108).

It was checked, with the western blotting analysis, whether tyrosine-phosphorylated RON (pTyr-wt/mRON) has been expressed in the parent cell line LIM1215 and the cell line R2 exhibiting secondary resistance to cetuximab. As a result, it was confirmed that pTyr-mRON was expressed in the cell line R2 exhibiting secondary resistance to cetuximab.

In addition, it was observed that the cell line R2 exhibiting secondary resistance to cetuximab weakly inhibited the mechanism of RTK through weak feedback on ERK signaling, and thus cell death was induced in response to the compound of Example 1 (left of FIG. 5). In addition, it was observed that mRON and β-catenin are bound to each other (right of FIG. 5).

### Experimental Example 4. Analysis of cell death induction using β-catenin

Each of an empty vector and human beta-catenin pcDNA3.1 (Plasmid #16828, Addgene), i.e., a vector into which β-catenin DNA is introduced, was transfected into the cell line R2 exhibiting secondary resistance to cetuximab, and then treatment with negative control (DMSO) or the compound of Example 1 was performed. Subsequently, all cells were collected when 48 hours had elapsed. Cell death induction efficacy was compared and analyzed using a trypan blue exclusion assay. In addition, using these samples, changes in expression of respective proteins were compared and analyzed with the western blotting method.

The cell line R2 exhibiting secondary resistance to cetuximab was caused to overexpress β-catenin. Then, treatment with the compound of Example 1 was performed, and cell death induction efficacy was observed. As a result, it was observed that cell death induction efficacy of the compound of Example 1 was reduced (FIG. 6).

In addition, the cell line R2 exhibiting secondary resistance to cetuximab was caused to overexpress β-catenin. Then, treatment with the compound of Example 1 was performed, and expressions of pTyr-mRON, p-ERK, β-catenin, c-myc, and cleaved caspase-3 were checked. As a result, it was observed that expression of pTyr-mRON was decreased by the compound of Example 1 regardless of β-catenin, and that expressions of p-ERK, β-catenin, and c-myc were decreased in the cell line transfected with the empty vector and were increased again when β-catenin was overexpressed. On the contrary, it was observed that expression of cleaved caspase-3 was decreased when β-catenin was overexpressed (FIG. 7).

### Experimental Example 5. Efficacy of compound of Example 1 on cell line exhibiting secondary resistance to cetuximab (in vivo)

5-Week-old female BALB/c nude mice were purchased and acclimatized for 1 week. Then, the cell line R2 exhibiting secondary resistance to cetuximab (5×10⁶ cells/mice) was diluted in PBS, and injected subcutaneously (100 µL) into the right dorsal side of the mice. The compound of Example 1 was orally administered when the tumor reached a size of about 100 mm³. The compound of Example 1 was administered once a day for 2 weeks, and tumor size and body weight were measured twice a week.

In the case of cetuximab, intraperitoneal administration was given twice a week. After completion of the 4-week experiment, the experimental animals were euthanized, and then the tumors were extracted and weighed. In addition, with respect to the tumor tissues, changes in expressions of pTyr-mRON, mRON, β-catenin, and cleaved caspase-3 were analyzed with immunohistochemical staining.

As a result, the dosages of cetuximab and the compound of Example 1, and the resulting tumor growth inhibition are as shown in Table 3 below:

**[Table 3]**

| | **Cetuximab** | **Example 1** | |
|---|---|---|---|
| **Dosage** | 40 mg/kg | 5 mg/kg | 15 mg/kg |
| **Tumor growth inhibition (TGI, %)** | 24.2±5.4 | 37.5±6.8 | 62.3±4.5 |

As shown in Table above, it was confirmed that even when treatment with a small dose of the compound of Example 1 was performed, a remarkably excellent tumor size inhibition effect was exhibited as compared with cetuximab (FIG. 8).

In addition, changes in expressions of pTyr-mRON, mRON, β-catenin, and cleaved caspase-3 were analyzed. As a result, it was confirmed that expressions of pTyr-mRON and β-catenin were decreased and expression of cleaved caspase-3 was increased only in the case of treatment with the compound of Example 1 (FIG. 9).

## Claims

1. A pharmaceutical composition for preventing or treating cancer associated with a V-Ki-ras2 Kirsten rat sarcoma viral oncogene homolog (KRAS) mutation, the composition comprising, as an active ingredient, a compound of Formula 1 or Formula 2 below or a pharmaceutically acceptable salt thereof,
wherein the cancer exhibits resistance to an epidermal growth factor receptor (EGFR)-targeted therapeutic agent, and the KRAS mutation is substitution of glycine, an amino acid residue, at position 13 in an amino acid sequence of SEQ ID NO: 1 with aspartic acid: 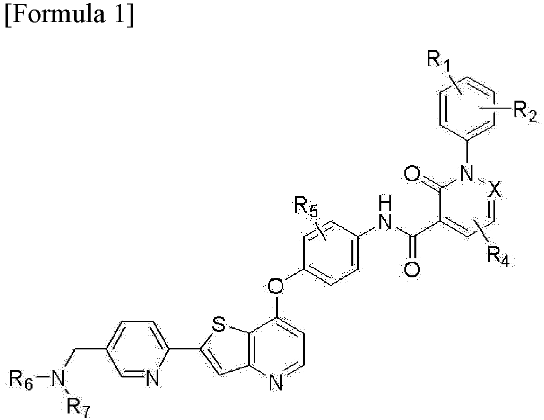
wherein, in Formula 1 above,
R₁ and R₂ are each independently H, halogen, C₁₋₁₀ alkoxy, or halo C₁₋₁₀ alkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ form a 4- to 10-membered heterocycle together with the N atom to which they are bonded, or R₆ is -C₂H₄-O-CH₃, and R₇ is H, methyl, or t-butoxycarbonyl; and
the heterocycle optionally further contains one or two heteroatoms selected from the group consisting of N, O, and S, in addition to the N atom to which R₆ and R₇ are bonded, and is unsubstituted or substituted with one or more substituents selected from halogen and C₁₋₆ alkyl, 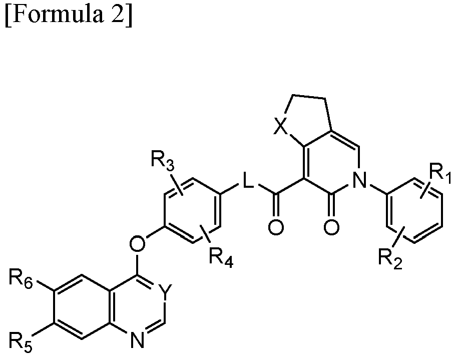
in the formula 2,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl, or 3- to 9-membered heterocycloalkyl,
X is O, S, -CH(-Rx)-, or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
Y is -N= or -CH=,
R₅ and R₆ are each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, diC₁₋₆ alkylcarbonylamino, C₁₋₆ alkylcarbonylamino, C₁₋₆ alkylamino, or C₁₋₆ alkylamino-C₁₋₆ alkoxy,
wherein R₅ and R₆ are each independently optionally substituted with 3- to 9-membered cycloalkyl; or 3- to 9-membered heterocycloalkyl,
the cycloalkyl or the heterocycloalkyl optionally has one or more substituents selected from the group consisting of halogen, oxo, cyano, hydroxy, hydroxy-C₁₋₆ alkyl, amino, diC₁₋₆ alkylamino, C₁₋₆ alkyl, C₁₋₆ alkoxy, and C₁₋₆ alkoxy-C₁₋₆ alkyl, and
the heterocycloalkyl contains 1 to 4 heteroatoms selected from the group consisting of N, O, and S.

2. The pharmaceutical composition of claim 1, the compound represented by Formula 1 is selected from the group consisting of:
4-ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3-carboxamide;
4-Ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide; and
4-ethoxy-N-{3-fluoro-4-[(2-{5-[(4-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

3. The pharmaceutical composition of claim 1, the compound represented by Formula 2 is N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide; or
N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide.

4. The pharmaceutical composition of claim 1, wherein the EGFR-targeted therapeutic agent is cetuximab, gefitinib, erlotinib, or panitumumab.

5. The pharmaceutical composition of claim 1, wherein the resistance to the EGFR-targeted therapeutic agent is associated with a recepteur d'origine nantais (RON) mutation.

6. The pharmaceutical composition of claim 5, wherein the RON mutation is RONΔ155 in which exons 5, 6, and 11 are deleted.

7. The pharmaceutical composition of claim 1, wherein the cancer is selected from the group consisting of breast cancer, lung cancer, stomach cancer, prostate cancer, uterine cancer, ovarian cancer, kidney cancer, pancreatic cancer, liver cancer, colorectal cancer, skin cancer, head and neck cancer, and thyroid cancer.

8. A use of the compound represented by Formula 1 or Formula 2 as defined in claim 1 or a pharmaceutically acceptable salt thereof, for preventing or treating cancer associated with a KRAS mutation,
wherein the cancer exhibits resistance to an EGFR-targeted therapeutic agent, and the KRAS mutation is substitution of glycine, an amino acid residue, at position 13 in an amino acid sequence of SEQ ID NO: 1 with aspartic acid.

9. A use of the compound represented by Formula 1 or Formula 2 as defined in claim 1 or a pharmaceutically acceptable salt thereof, for preparation of a medicament for preventing or treating cancer associated with a KRAS mutation,
wherein the cancer exhibits resistance to an EGFR-targeted therapeutic agent, and the KRAS mutation is substitution of glycine, an amino acid residue, at position 13 in an amino acid sequence of SEQ ID NO: 1 with aspartic acid.

10. A method for preventing or treating cancer associated with a KRAS mutation, the method comprising administering the compound represented by Formula 1 or Formula 2 as defined in claim 1 or a pharmaceutically acceptable salt thereof to a subject in need thereof,
wherein the cancer exhibits resistance to an EGFR-targeted therapeutic agent, and the KRAS mutation is substitution of glycine, an amino acid residue, at position 13 in an amino acid sequence of SEQ ID NO: 1 with aspartic acid.

11. A method for providing information on an anticancer therapeutic agent, the method comprising:
identifying a KRAS mutation in an individual who exhibits resistance to an EGFR-targeted therapeutic agent; and
providing information that the compound represented by Formula 1 or Formula 2 as defined in claim 1 or a pharmaceutically acceptable salt thereof is suitable for anticancer therapy when a KRAS mutation, in which glycine, an amino acid residue, at position 13 in the amino acid sequence of SEQ ID NO: 1 is substituted with aspartic acid, is found.

12. The method of claim 11, wherein the EGFR-targeted therapeutic agent is cetuximab, gefitinib, erlotinib, or panitumumab.
